# EUROPEAN PATENT APPLICATION

(11) **EP 1 857 064 A1**
(43) Date of publication of application: **21.11.2007**
(21) Application number: 06009995.9
(22) Date of filing: 15.05.2006
(51) Int. Cl.: A61B 17/70, A61B 17/86

(54) **Surgical screw system**

(71) Applicant: Biomet Spain Orthopaedics S.L., Paterna (Valencia) (ES)
(72) Inventor: Albert Alarcon, Miguel, CP 46190, Ribarroja del Turia, Valencia (ES); Marco Linares, José Maria, CP 46014, Valencia (ES); Sierra Aparici, Alfredo, CP 46019, Valencia (ES)
(74) Representative: Bauch-Koepe, Katharina Anna

(57) **Abstract**

The present invention relates to a system of means allowing a fixation of one or more implantation rods to at least parts of the spinal column of a vertebrate, comprising at least one implantation rod and at least one of the following means: a sacrum plate; at least one polyaxial screw; at least one monoaxial expansive screw; at least one monoaxial screw; at least one multidirectional coupler; at least one multidirectional offset coupler; at least one side loading expansive screw; at least one transverse connector; at least one hook; at least one closed parallel connector; at least one coaxial connector; at least one multidirectional double coupler; at least one injection screw. The invention also relates to a method for applying the system to a vertebrate and to the use of said system.

## Description

The present invention relates to a surgical screw system for use with implantation rods and relates to using such a system. In particular, the present invention relates to a surgical screw system in connection with rods for achieving an immobilization of the spinal column in cases where this is considered necessary for any reason, and relates to a method using a surgical screw system in connection with immobilizing rods. The implantation rods can be introduced either through posterior or through anterior approach.

The spinal column of a human adult consists of at least 24 discrete bones called vertebrae which, in their sequence, together form the spinal column. According to the location in the human body where the vertebrae are located, 7 cervical vertebrae, 12 thoratic vertebrae, 5 lumbar vertebrae and at least 1 sacral vertebra are distinguished. The vertebrae form a highly flexible structure of the spinal column which allows a high degree of curvature and twist in almost every direction. In addition, the spinal column houses, and thereby protects, main cords of the nervous system and of circulatory vessels.

Genetic and development irregularities, trauma, chronic stress as well as tumors and other diseases may lead to pathologic conditions of the spinal column which deteriorate the flexibility and range of motion of the spinal column. Such deteriorations may also occur as a result of diseases of the nervous system housed within the spinal column. There were developed numerous systems or implants which allow an immobilization of the spinal column in order to improve such conditions or cure such diseases. These implants may comprise, and in cases of posterior implants generally comprise, one or two rod(s) which, in a surgical procedure, may be attached to the spinal column either by hooks coupling to the lamina or connecting to the transverse processes or by screws inserted through the pedicles so as to be aligned along at least a part of the spinal column and to stabilize it against movements with the aim of supporting the curing process.

The document WO-A 02/054,966 discloses a polyaxial pedicle screw assembly which incorporates a concave portion on a receiver which mates with a convex surface on a head of the screw so as to form a ball joint. The radius of at least a portion of the concave surface is less than the radius of the mating convex portion thereby to create an interference fit.

The document WO-A 01/22,893 relates to a surgical screw system for use with implantation rods. The screw system includes a screw member and a receiver member adapted to each other with respect to their geometrical shapes. An implantation rod is secured to a part of the receiver member by means of a locking device applying a torque upon the rod when positioned in the receiver member.

The document EP 1 254 639 relates to a dorsolumbar and lumbosacral vertebrae fixing system which is based on a connector in the form of a clamp that is provided with a hollow multidirectional swivel joint which is traversed by a rod. The clamp also has a circular or elliptical hole through which the end of the fixing element to the vertebra is fixed. Said element may be a hollow expansion screw that opens against the spongy element when a pin is inserted inside or when a hook that is coupled to the vertebra in the laminar or pedicular area is inserted.

In order to make the handling of orthopedic devices in the course of surgery easier and more reliable, and in order to provide systems maintaining a long durability once installed, it was desired to provide improvements of surgical screw systems proposed by the prior art.

Surprisingly, it was found that the systems of the prior art can be improved substantially, and an excellent, reliable and durable lock of the rods can be obtained easily in a surgical process by using the surgical screw system of the present invention. In addition, the method of using the present system provides a reliable and durable way for locking the implantation rods and can easily be implemented in the course of surgery.

Hence, the invention relates to a system of means allowing a posterior or anterior fixation of one or more implantation rods to at least parts of the spinal column of a human, comprising at least one implantation rod and at least one of the following means: a sacrum plate; at least one polyaxial screw; at least one monoaxial expansive screw; at least one monoaxial screw; at least one multidirectional coupler; at least one multidirectional offset coupler; at least one side loading expansive screw; at least one transverse connector; at least one hook; at least one closed parallel connector; at least one coaxial connector; at least one multidirectional double coupler; at least one injection screw.

Preferred embodiments of the invention are claimed in claims 2 to 8.

The invention also relates to the use of the system in detail described below for immobilizing at least parts of the spinal column of a human. Such immobilization and, hence, use of the invention may occur in a large number of diseases and conditions of a human, which include, but are not restricted to traumatic conditions or traumas as, for example, fractures, dislocations, disk herniation and numerous other injuries causing instability of the vertebra; degenerative diseases and conditions, which may be either acute or chronic processes, for example, but not restricted to, spondylolysis; inflammatory diseases and conditions which may be either acute or chronic inflammatory conditions and often are, but are not restricted to, spondylarthritis, spondylitis and those diseases of rheumatic nature; tumor diseases and conditions which may include benign and malign tumors; and congenital pathologies which are large in number and are of greatly varied origin, the most frequent example being spondylolisthesis.

Preferred uses are claimed in claims 10 to 12.

The invention also relates to a method of fixing at least one implantation rod to at least parts of the spinal column of a human, said method comprising the steps of
- selecting at least one implantation rod suitable in diameter(s) and length(s) for the intended implantation;
- either drilling at least one hole into at least two vertebrae, or preparing the space around the laminae or vertebral pedicle, at locations allowing a fixation of said at least one implantation rod to the spinal column at a desired position;
- inserting at least one, preferably at least two, of the following means:
   ■ at least one sacrum plate;
   ■ at least one polyaxial screw;
   ■ at least one monoaxial expansive screw;
   ■ at least one monoaxial screw;
   ■ at least one side loading screw;
   ■ at least one side loading expansive screw;
   ■ at least one injection screw;
- into the hole(s) drilled
- and/or fixing to the space prepared around the laminae or vertebral pedicle
   ■ at least one hook
- surrounding said pedicle or lamina; and
- fixing to any one or more of the side loading screw or side loading expansive screw or injection screw any other suitable screw at least one of
   ■ at least one multidirectional coupler;
   ■ at least one multidirectional offset coupler;
   ■ at least one multidirectional double coupler;
- so as to allow a fixation of said at least one implantation rod to said spinal column; and
- optionally further stabilize the arrangement of the above means by the application, between at least two of said implantation rods, of at least one of
   ■ at least one closed parallel connector;
   ■ at least one coaxial connector;
   ■ at least one transverse connector.

Preferred embodiments of said method are claimed in claims 14 to 21.

The invention is furtheron described in detail by referring to Figures, wherein
Figures 1A, 1B and 1C show three-dimensional perspective, elevational front and elevational side views, respectively, of the complete system for the fixation of at least one implantation rod according to the invention, said views being views to theoretical systems with a plurality of fixation means.
Figures 2A, 2B and 2C show coaxial connectors in accordance with the present invention for connecting implantation rods having equal or different diameters.
Figures 3A, 3B, 3C and 3D show three-dimensional perspective, elevational, top and sectional views, respectively, of closed parallel connectors in accordance with the present invention for connecting implantation rods having equal or different diameters.
Figures 4A to 4E show elevational front, elevational side, three-dimensional perspective, lateral and sectional views, respectively, of the hook part to be used in connection to the transverse connector as shown in Figure 5.
Figures 5A to 5F show three-dimensional perspective views of transverse connectors in accordance with the present invention for connecting non-parallel implantation rods.
Figures 6A to 60 show the top locking system of the present invention in its different embodiments.
Figures 7A to 71 show the sacrum plate including the top locking system in accordance with the present invention.
Figures 8A to 8D show three-dimensional perspective, sectional and elevational side views, respectively, of a monoaxial screw according to the invention, said monoaxial screw being provided with a tulip head suitable for the top locking system according to the invention for a fixation of an implantation rod.
Figures 9A to 9C show three-dimensional perspective and sectional views of a monoaxial expansive screw of the invention having a tulip head for top locking an implantation rod according to another embodiment of the invention.
Figures 10A to 10B show an explosion view and a three-dimensional perspective view of a multidirectional coupler of another embodiment of the invention, while Figure 10C shows the characteristics of the multidirectional coupler component, and Figure 10D represents a preferred embodiment of a side loading screw. Figures 10E to 10I show combinations of side loading screws with one or two implantation rods in perspective side elevational or top elevational views.
Figures 11A to 11B show an explosion view and a three-dimensional perspective view of a multidirectional offset coupler of another embodiment of the invention, while Figure 11C shows an elevational side view of the multidirectional offset coupler, and Figure 11 D shows the characteristics of the multidirectional offset coupler component.
Figures 12A to 12C show the pin insertion sequence for the side loading expansion screw of the present invention.
Figures 13A and 13B show three-dimensional perspective and elevational side views of a hook of the present invention provided with the top locking system for connecting the hooks to an implantation rod.
Figures 14A to 14G show a double multidirectional coupler according to another embodiment of the present invention.
Figures 15A to 15F show views of an injection screw in accordance with another embodiment of the invention.
Figures 16A and 16B show side sectional and perspective views, respectively, of a two-rod anterior connector of the invention.

The detailed description which follows, and the Figures, are intended to only exemplify the invention and are given for a better understanding of the invention. It is not intended that the subsequent description of the preferred embodiments of the invention, and the Figures as well, serve a limitation of the invention, but are given as non-limiting examples of preferred embodiments thereof.

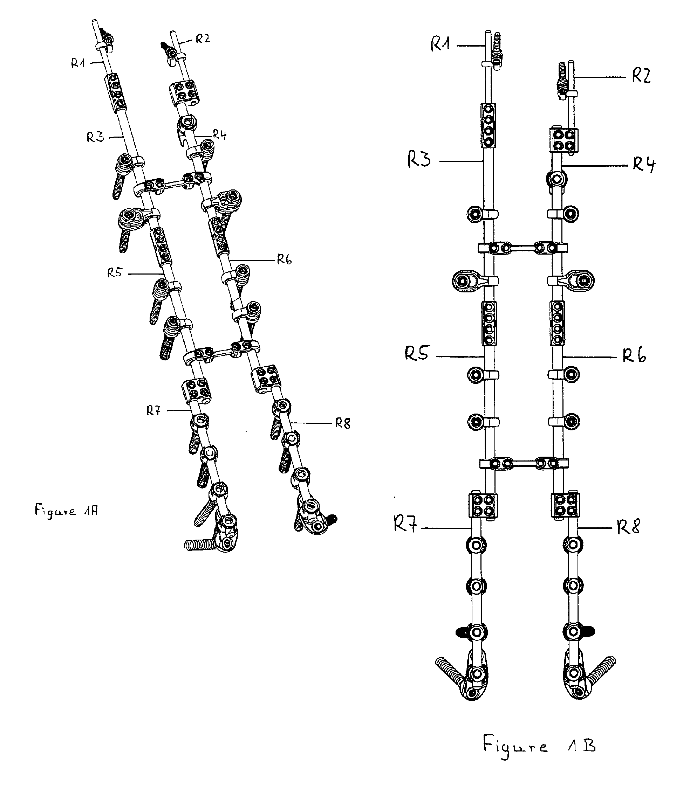

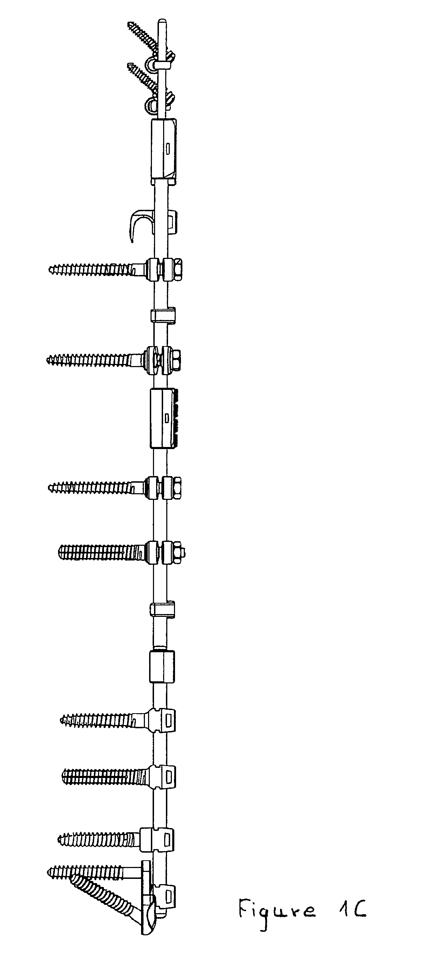

In accordance with the invention as generally shown in Figure 1, the system of allowing a fixation of at least one implantation rod R1, R2, R3, ... to at least parts of the spinal column of a human comprises at least one implantation rod.

The term "fixation", as used in the present description and in the claims, intends to describe the process by which - for whatever reasons described below in detail - at least one implantation rod is adapted to the spinal column of a human or at least a part thereof and, for at least a transient time, closely and either rigidly or flexibly connected to said (part of the) spinal column in a posterior or anterior manner allowing the implantation rod to exert an at least partly immobilizing force to said (part of the) spinal column. A rigid implantation rod is used in cases where a bone fusion has to be achieved, while a flexible implantation rod is used in cases where motion preservation has to be achieved. The connection between the implantation rod(s) R1, R2, R3, ... is achieved by the system of means of the present invention.

The term "to at least a part of the spinal column", as used in the present description and in the claims, intends to describe that the implantation rod(s) may be fixed to the spinal column of a human along its whole length, i. e. from the cervical vertebrae and along the thoracic vertebrae and along the lumbar vertebrae down to the sacral vertebrae, or they may be fixed to only a part of the spinal column, e. g. beginning from the (or any of the) thoracic vertebrae to the (or any of the) lumbar vertebrae or from the (or any of the) lumbar vertebrae to the (or any of the) sacral vertebrae. A fixation may be achieved over two, three, four or any desirable larger number of vertebrae consecutive in the spinal column and beginning, or ending, at any vertebra needed. Alternatively, a fixation may be achieved for two, three, four or any desirable number of vertebrae which are not in consecutive order at the spinal column but achieves a bridging of one or several vertebrae which need no immobilization.

The term "at least one implantation rod", as used in the present description and in the claims, intends to describe that the system of the present invention may use one single implantation rod R1 having either a uniform diameter or a diameter changing along the implantation rod (e. g. - not restricting the invention - having a larger diameter in areas where larger forces are applied and having a smaller diameter in areas where smaller forces are applied). However, in a similar way, the term "at least one implantation rod" includes two implantation rods R1, R2, three implantation rods R1, R2, R3, four implantation rods R1, R2, R3, R4 and also any larger number of implantation rods, as needed in a specific situation of, for example, immobilizing the spinal column of a human. In cases of two and more implantation rods R1, R2, R3, ..., these two or more rods may have the same diameter or may have different diameters, as needed in a specific situation. Two or more implantation rods R1, R2, etc. may be arranged in parallel or may be arranged side by side, but including a certain angle, or may be arranged in line, i. e. one above the other end by end or, alternatively, with a gap of any width between their ends, or may be arranged even in a coaxial way; any of arrangements conceivable by a skilled person and suitable to achieve the desired aim can be selected and is included by the present invention.

The term "side loading screw" as used in the present specification and in the claims refers to a pedicle type screw with a lateral connector for a fixation to an implantation rod R1, R2 etc.. In contrast thereto, the term "direct loading screw" as used in the present specification and in the claims refers to a pedicle type screw with a direct fixation to an implantation rod R1, R2 etc. through an U-shaped bearing.

In a preferred embodiment of the invention, the system comprises at least two implantation rods R1, R2, which are arranged in a parallel relationship to each other. In such a case, these (at least) two implantation rods R1, R2 may have the same diameter and or may have the same length, but they may also be different in diameter and/or length.

In another preferred embodiment of the invention, the system comprises at least two implantation rods R1, R3, which are arranged in a coaxial relationship to each other. In such a case, these (at least) two implantation rods R1, R3 may have the same diameter and or may have the same length, but they may also be different in diameter and/or length.

In another preferred embodiment of the invention which - as the above two other embodiments - are exemplary only and do not restrict the invention, the system comprises at least two parallel implantation rods (R1, R2; R3, R4) and at least two coaxial implantation rods (R1, R3; R2, R4). Also in this case, these respective two parallel/coaxial implantation rods R1, R2, R3, R4, ... may have the same diameter and or may have the same length, but they may also be different in diameter and/or length.

In cases of using more than one implantation rod R1, R2, R3, ..., it is a preferred embodiment of the invention that at least two implantation rods are connected to each other in order to enhance the stabilization of the whole fixation system. In an even more preferred embodiment, two or more than two coaxial implantation rods R1, R3, R5, ... are connected by means of one or more than one coaxial connector 1.11.. The coaxial connector may connect two coaxial implantation rods R1, R3, ... of the same length and/or diameter or may connect two coaxial implantation rods R1, R3, ... of different lengths and/or diameters.

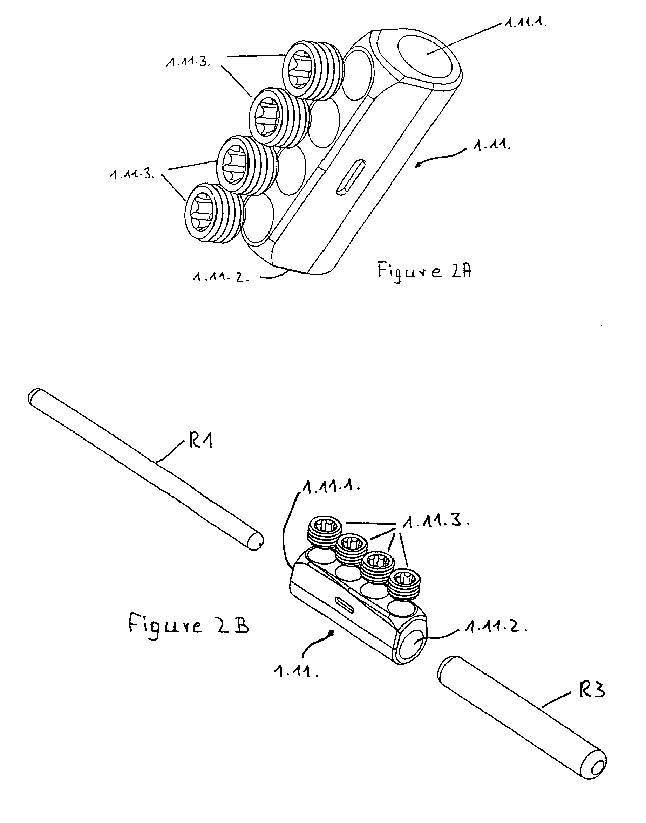

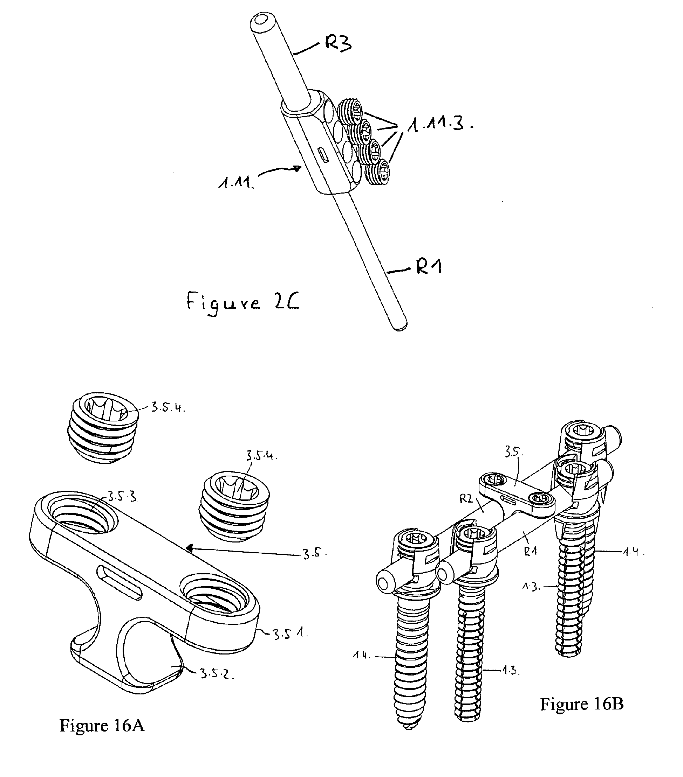

Particularly preferred examples of the coaxial connectors 1.11. are shown in Figures 2A, 2B and 2C. Figure 2A shows the general embodiment of a coaxial connector 1.11. where one hole 1.11.1. and 1.11.2. at either ends of the connector 1.11. allows an insertion of two implantation rods R1, R3 on different sides of the connector. After inserting the two implantation rods R1, R3, both rods are fixed in their inserted position by two nuts 1.11.3. each, i. e. in that case by four nuts 1.11.3.. By fixing the two rods R1, R3 in their position inserted into the coaxial connector 1.11., a solid and rigid connection between them is established. Figure 2B shows an exploded view of two (different diameter) coaxial implantation rods R1, R3, being inserted into the respective holes 1.11.1. and 1.11.2. of the coaxial connector 1.11., with the four fixation nuts 1.11.3. removed from their positions, and Figure 2C shows the assembly of two (different diameter) coaxial implantation rods R1, R3, the coaxial connector 1.11. and the four fixation nuts 1.11.3. to be inserted into their positions and to be fixed, hence being prepared for creating a solid and rigid connection between the two rods.

The fixation nuts 1.11.3. may have means for fixation generally known in this technical field. As examples, they may have a lower end (contacting the implantation rod(s)) having a conical tip and allowing a punctual contact in the fixed state. As another example, they may have a hexagon or hexalobe allowing to engage a corresponding operation tool in the step of establishing the fixation.

Similar arrangements as shown in Figure 2C can also be seen in Figures 1A, 1 B and 1C, respectively.

In another particularly preferred embodiment, two parallel implantation rods are connected by one or more than one closed parallel connector 1.10.. The closed parallel connector 1.10. may connect two parallel implantation rods R1, R2, ... of the same length and/or diameter or may connect two parallel implantation rods R1, R2, ... of different lengths and/or diameters.

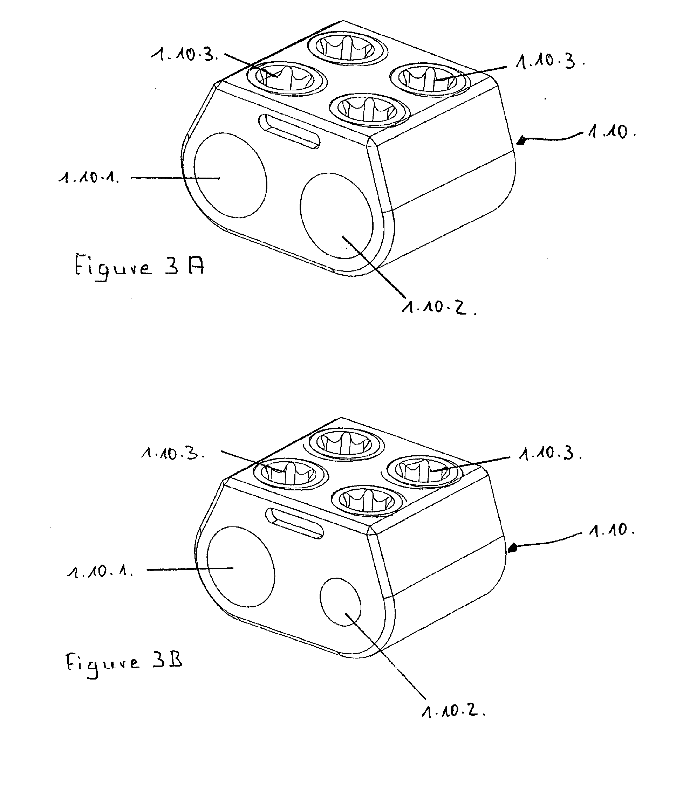

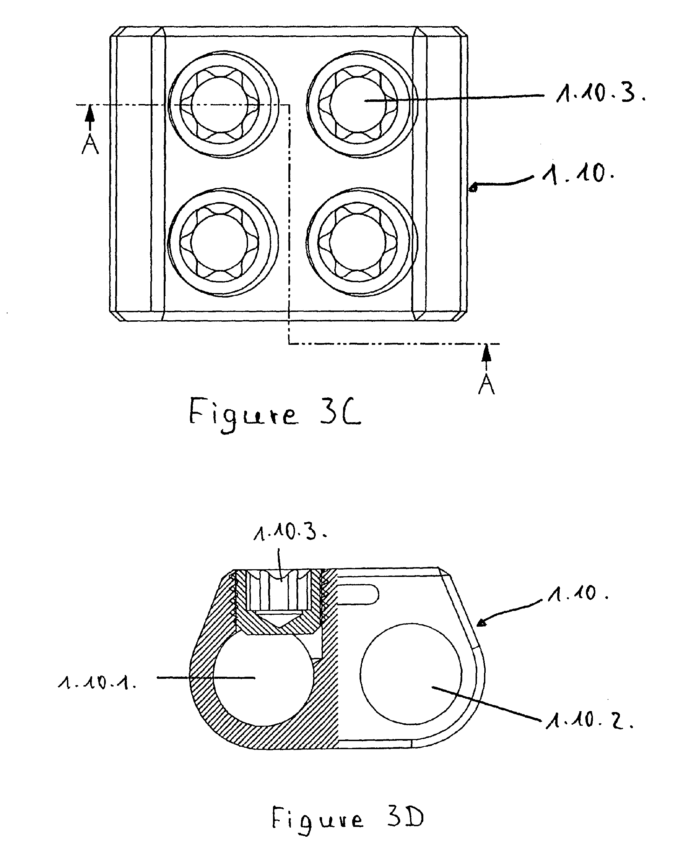

Particularly preferred examples of the closed parallel connectors 1.10. are shown in Figures 3A, 3B, 3C and 3D. Figure 3A shows a perspective view of the general embodiment of a closed parallel connector 1.10. where two holes 1.10.1. and 1.10.2. at one end of the connector 1.10. allow an insertion of two implantation rods R1, R3. After inserting the two implantation rods R1, R3, both rods are fixed in their inserted position by two nuts 1.10.3. each, i. e. in that case by four nuts 1.10.3.. By fixing the two rods R1, R3 in their position inserted into the closed parallel connector 1.10., a solid and rigid connection between them is established. Figure 3B shows a perspective view of a closed parallel connector 1.10. similar to that one shown in Figure 3A, but being adapted to connect two implantation rods R1, R3 having different diameters by inserting them into holes 1.10.1. and 1.10.2., respectively, and closing the fixation nuts 1.10.3., hence creating a solid and rigid connection between the two rods R1, R3. Figure 3C shows a top view of the closed parallel connector 1.10., and Figure 3D shows a sectional view along the line A - A in Figure 3C. As can be seen from Figure 3D, the closed parallel connector shown is adapted to connect implantation rods R1, R3 of equal diameter.

The fixation nuts 1.10.3. may have means for fixation generally known in this technical field. As examples, they may have a lower end (contacting the implantation rod(s)) having a conical shape and including a tip and allowing a punctual contact in the fixed state. As another example, the may have a hexagon or hexalobe allowing to engage a corresponding operation tool in the step of establishing the fixation.

Arrangements of parallel implantation rods R1, R3 connected by closed parallel connectors 1.10. can also be seen in Figures 1A, 1B and 1C, respectively.

In another preferred embodiment of the invention which may be realized separately from the afore-mentioned embodiments or may be realized together with them, at least two parallel implantation rods R1, R2 are connected by means of a open parallel connector 3.5.. The open parallel connector 3.5. is suitable to connect, and maintain, two parallel implantation rods R1, R2 in a strictly parallel manner.

Figures 16A and 16B show perspective and sectional views, respectively, of a open parallel connector 3.5. which may be used in transversely connecting two implantation rods extending exactly in parallel. The open parallel connector 3.5. has a shape similar to an anvil, i. e. consists of an upper plate 3.5.1. from the lower side of which two hook-like means 3.5.2. are extending downwards which hooks are arranged opposite to each other. When used as a connector, the latter opposite hook-like means 3.5.2. serve to receive the two parallel implantation rods, which rods are fixed by means of two cylindrical threaded fixation nuts 3.5.4. which are threaded into threaded holes 3.5.3. provided in the upper plate 3.5.1. in a manner parallel to each other. Advantageously, the transversely connecting open parallel connector provides a safe and rigid parallel fixation of two implantation rods R1, R2.

The fixation nuts 3.5.4. may have means for fixation generally known in this technical field. As examples, they may have a lower end (contacting the implantation rods R1, R2) having a conical shape and including a tip and allowing a punctual contact in the fixed state. As another example, they may have a hexagon or hexalobe allowing to engage a corresponding operation tool in the step of establishing the fixation.

In another preferred embodiment of the invention which may be realized separately from the afore-mentioned embodiments or may be realized together with them, at least two parallel implantation rods R1, R2 are connected by means of a transverse connector 1.8.. The transverse connector 1.8. is suitable to connect parallel implantation rods R1, R2 and, moreover, also allows to achieve a proper fixation of rods which cannot be exactly parallel for constitutional reasons. This gives more freedom when providing a fixation of non-parallel implantation rods to each other and additionally allows, for the first time, to insert a connecting device for two implantation rods through a vertical direction.

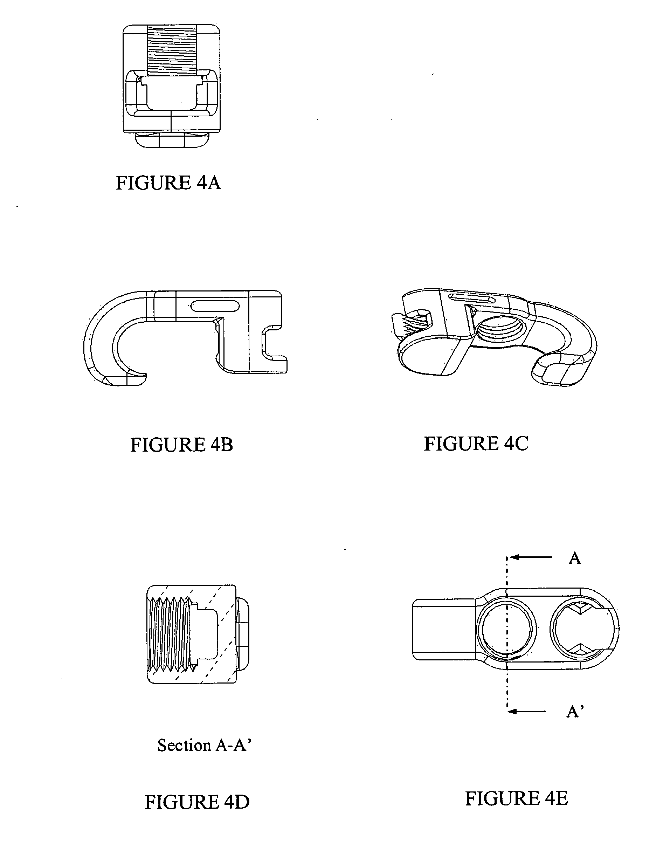

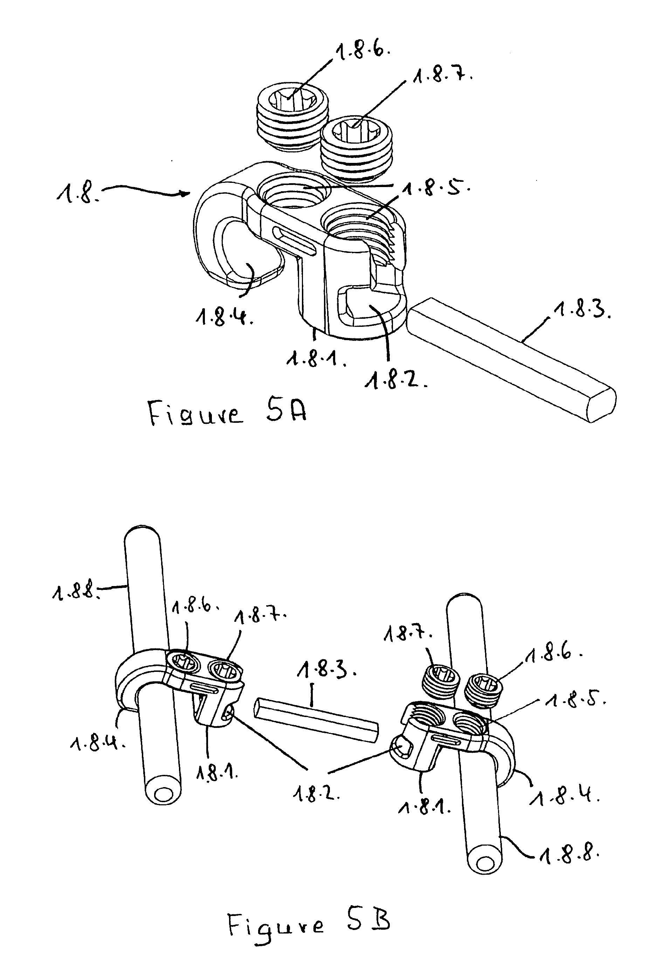

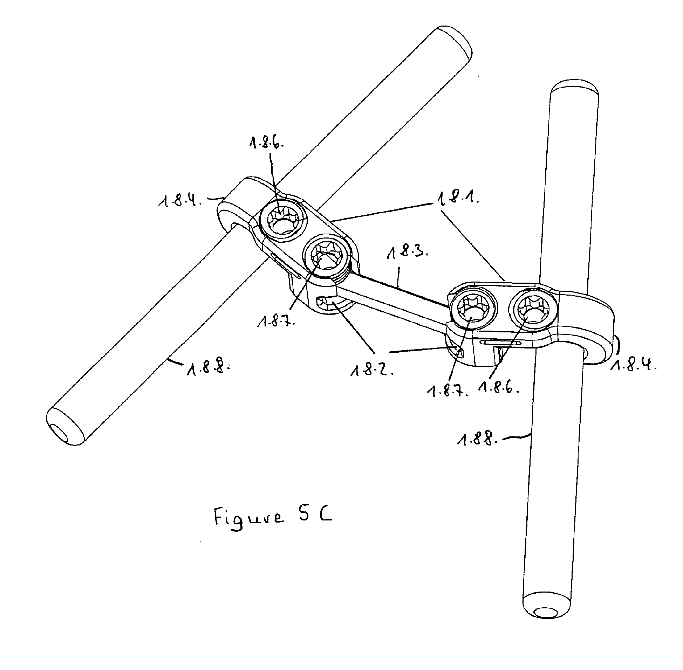

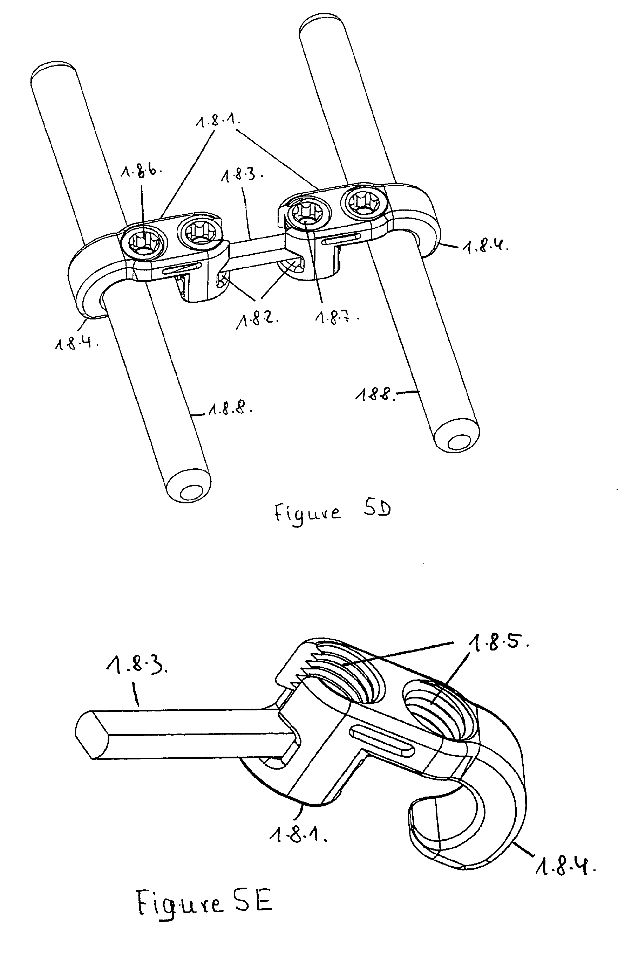

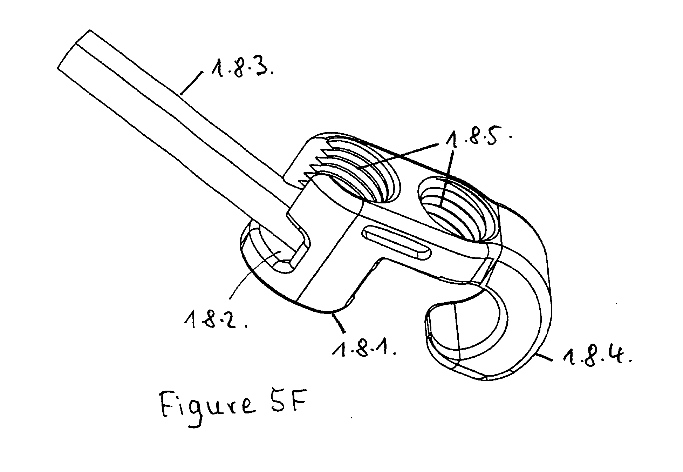

Figures 4A, 4B, 4C, 4D and 4E show elevational front, elevational side, lower three-dimensional perspective, lateral and sectional views, respectively, of a hook part to be used in connection to the transverse connector as shown in Figure 5. The hook part comprises a hemi-cylindrical main body from which a hook element is extending on the upper side, said hook element having a hook bent downwards. The hook element is intended to be engaged to an implantation rod. For such a purpose, the hook element comprises in its upper part a threaded hole into which a screwing fixation nut is to be inserted and screwed, the lower tip or lower surface of which presses the implantation rod towards the end portion of the hook. The hemi-cylindrical main body of the hook part has a threaded hole extending parallel to the threaded hole of the hook element, and additionally has, on its cylinder wall side and perpendicular to the threaded hole, a channel for inserting a transverse rod allowing to connect two hook parts and thereby creating the transverse connection between two implantation rods. In order to fix the transverse rod in said channel, a screwing fixation nut is screwed into the threaded hole in the hemi-cylindrical main body of the hook part, the lower tip or surface of which fixes the transverse rod in said channel.

Figures 5A to 5F show three-dimensional perspective views of the transverse connector 1.8. of the present invention. Figure 5A shows a perspective view of the transverse connector 1.8. in an exploded view, wherein 1.8.1. indicates the transverse connector main body having a channel 1.8.2. for inserting the transverse rod 1.8.3., a hook 1.8.4. bent downwardly for engaging with the implantation rod 1.8.8. to be connected to and two threaded holes 1.8.5. for inserting and screwing fixation nuts 1.8.6. and 1.8.7. fixing the transverse rod 1.8.3. and an implantation rod 1.8.8.. Figure 5B shows two transverse connector main bodies 1.8.1. engaged (right side) and fixed (left side) to respective implantation rods 1.8.8., and a transverse rod 1.8.3. having the appropriate length and ready to be inserted into the respective channels 1.8.2. of the transverse connector main bodies 1.8.1. and then to be fixed with respective fixation nuts 1.8.7.. Figures 5C and 5D, respectively, show the final arrangement of the transverse connectors 1.8. in cases of parallel or non-parallel implantation rods 1.8.8.. Figures 5E and 5F show the advantageous flexibility, around fixed maximum angles, of the transverse rod 1.8.3. when inserted into the channel 1.8.2. of the transverse connector main body 1.8.1..

The main advantages of this transverse connection are a surprising flexibility in cases where the implantation rods 1.8.8. are either exactly parallel or are arranged including a certain angle, where the physiological conditions require such a deviation from the parallel arrangement. In addition, the surgeon may achieve the possibility to introduce the transverse connector 1.8. in a vertical direction. Finally, the fixation step of the transverse connector main body 1.8.1. to the implantation rods 1.8.8., on the one hand, and to the transverse rod 1.8.3. can be performed independently, thus allowing the surgeon an adjustment of the fixation before the rigid position of all parts relative to each other is fixed. In addition, the transverse rod 1.8.3. may be adjusted in length to the distance of the two connector main bodies 1.8.1. needed to achieve a fixation of the (more or less) parallel implantation rods 1.8.8. in the desired position.

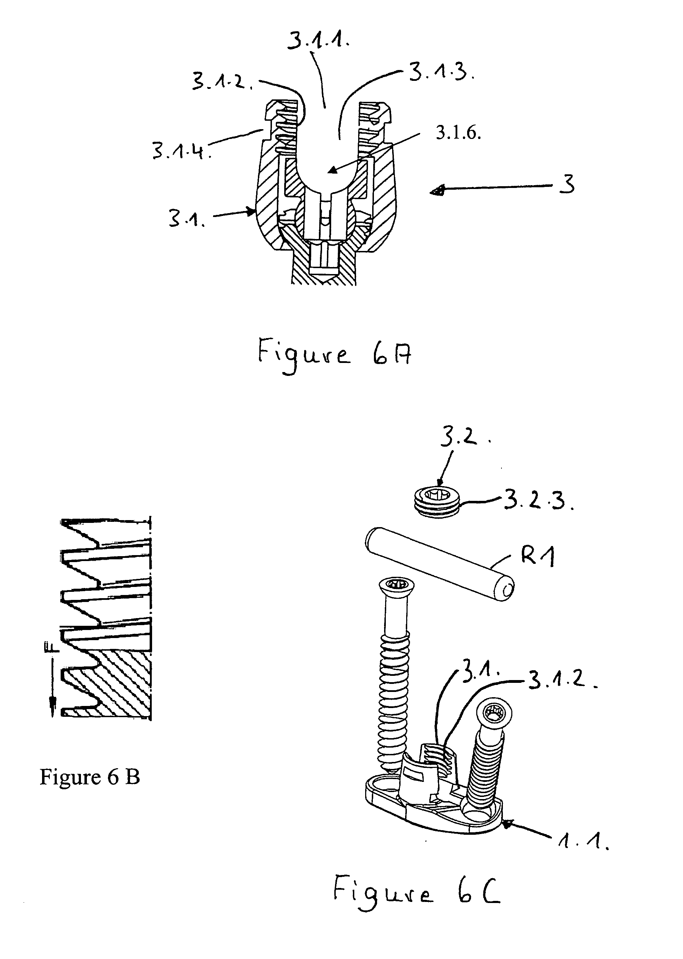

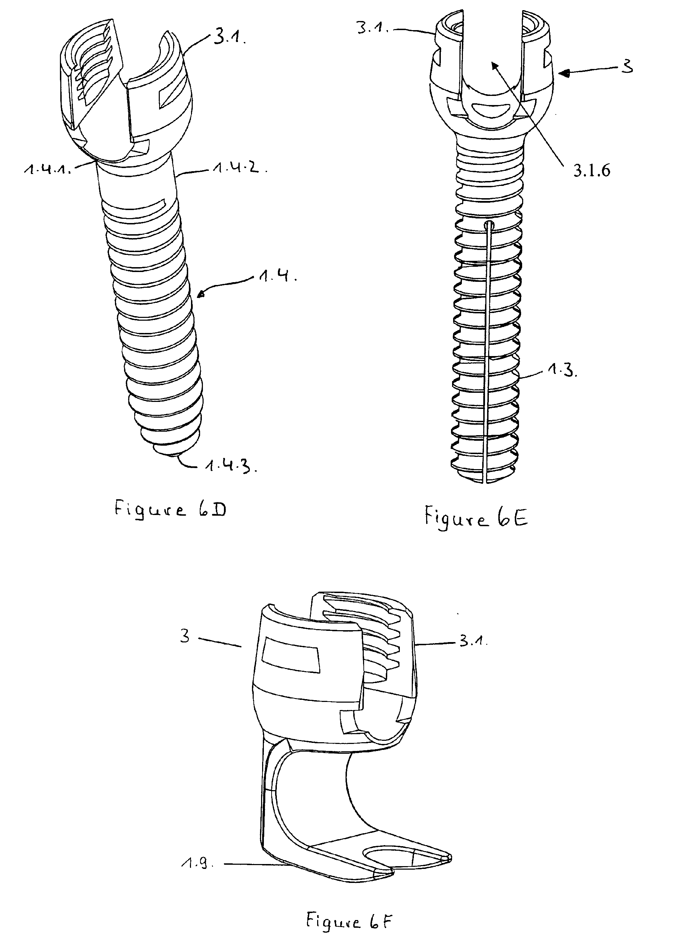

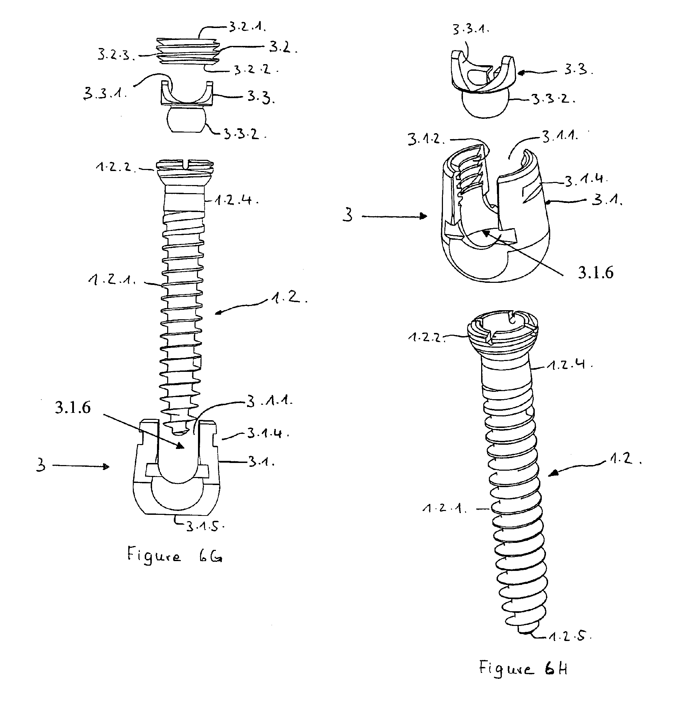

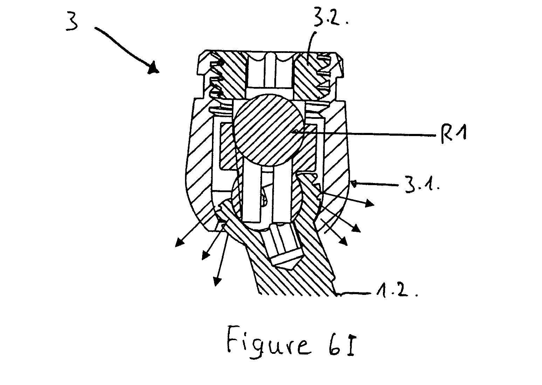

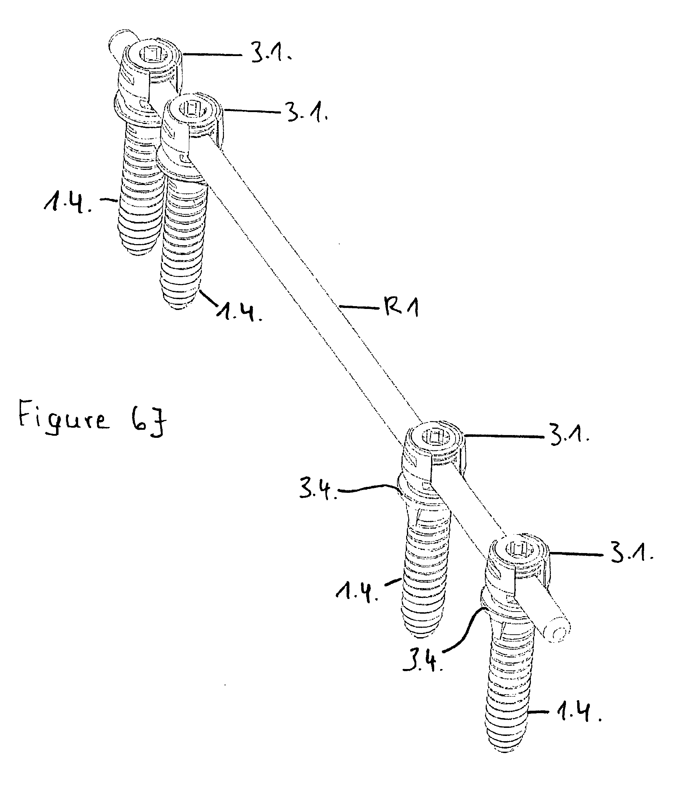

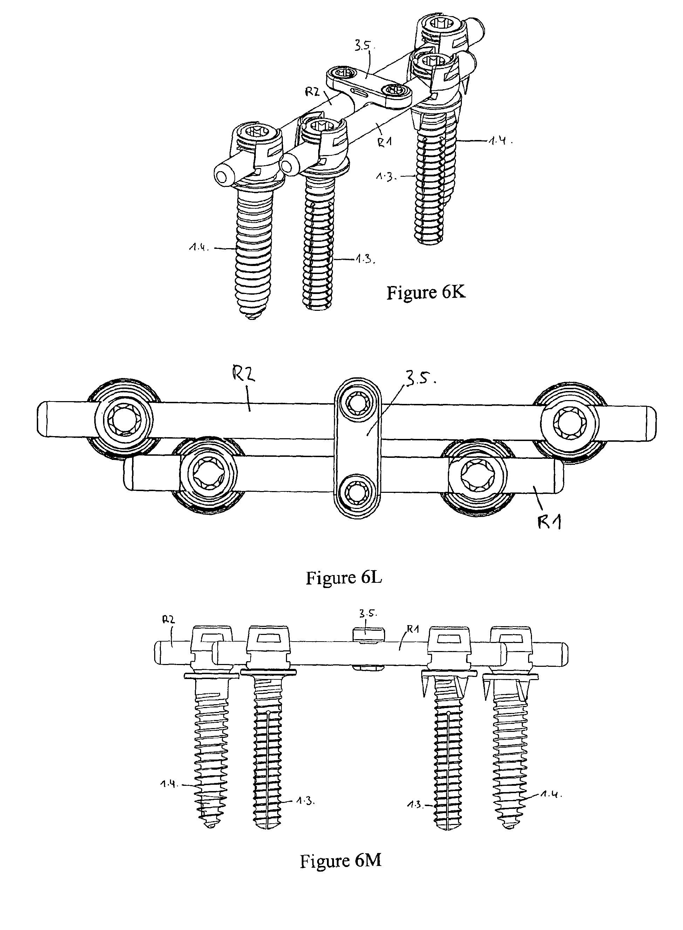

Reference is now made, in general, to Figure 1A to 1C. In another preferred embodiment of the invention, the system of means allowing a fixation of at least one implantation rod R1, R2, R3, ... to at least parts of the spinal column of a human comprises one or several parts thereof, in particular the sacrum plate 1.1. and/or the polyaxial screw 1.2. and/or the monoaxial expansive screw 1.3. and/or the monoaxial screw 1.4. and/or the hook 1.9., in a way that one or two or more or all of them are provided with a top locking system 3 as exemplified in Figures 6A to 60, said top locking system 3 comprising:
- a tulip-shaped head 3.1, having an upper orifice 3.1.1. having an inner trapezoidal thread 3.1.2., an inner chamber 3.1.3. having a diameter allowing to house the implantation rod R1, R2, R3, ...., a large U-shaped indentation 3.1.6. on two opposite sides of the tulip-shaped head 3.1., a lateral slot 3.1.4. on the respective other two opposite sides outside of the tulip-shaped head 3.1., and, in the case of the tulip-shaped head 3.1. for a polyaxial screw, a lower orifice 3.1.5. smaller in diameter than the upper orifice 3.1.1. thereby forming an annular base; and
- a cylindrical threaded nut 3.2. having an upper and a lower surface 3.2.1., 3.2.2. and an outer trapezoidal thread 3.2.3. fitting with the inner trapezoidal thread 3.1.2. of the tulip-shaped head 3.1. and intended to be screwed into the inner trapezoidal thread 3.1.2. of the tulip-shaped head 3.1. so as to fix the implantation rod R1, R2, R3, ... housed in said inner chamber 3.1.3. by a contact between said implantation rod R1, R2, R3, ... and said lower surface 3.2.2. to the annular base;
- said top locking system 3 being provided on the respective sides of said sacrum plate 1.1. and/or polyaxial screw 1.2. and/or monoaxial expansive screw 1.3. and/or monoaxial screw 1.4. and/or hook 1.9. which are opposite to the respective threaded screw sides or blades thereof.

Hence, the top locking system 3 of the invention is a system by which a simplified and more flexible fixation of the implantation rod(s) R1, R2, R3, ... to the spinal column can be achieved. This can best be seen from Figures 6A to 60. Figure 6A shows a cross-sectional view of a polyaxial tulip-shaped head 3.1.. The head has an upper orifice 3.1.1. arranged substantially perpendicular to the axis of the tulip-shaped head 3.1.. The inner wall of said orifice 3.1.1. is provided with a thread 3.1.2. which is trapezoidal in shape, as shown in Figure 6B. Due to the trapezoidal shape of the thread 3.1.2., the fixation forces advantageously exert no horizontal component and are directed vertically in the process of fixing the tulip-shaped head 3.1. to the implantation rod R1, R2, R3, ... as can, for example, be seen schematically from the arrows in Figure 61.

Figure 6C shows one application example of the top locking system 3. The sacrum plate 1.1., the structure and function of which is described in more detail below, may be provided, in a preferred embodiment of the invention, with the top locking system 3 comprising the tulip-shaped head 3.1. and the cylindrical threaded nut 3.2.. As can be seen from Figure 6C, the two sacrum screws, once guided through the sacrum screw holes in the sacrum plate 1.1. and screwed to the sacrum bone, allow an access of the tulip-shaped head 3.1. of the top locking system provided on the upper side of the sacrum plate (i. e. the side of the plate opposite to the place where the sacrum screws are screwed into the sacrum bone (not shown in Figure 6C)). An implantation rod R1 is inserted into the U-shaped indentations 3.1.6. on both sides of, and crossing the tulip-shaped head 3.1.. Then, the cylindrical threaded nut 3.2. is inserted into the upper orifice 3.1.1. of the tulip-shaped head 3.1., and the outer thread 3.2.3. of the cylindrical threaded nut 3.2. engages the trapezoidal thread 3.1.2. of the inner wall of the upper orifice 3.1.1. of the head 3.1.. Once in contact with the implantation rod R1, the cylindrical threaded nut 3.2. rigidly fixes the implantation rod R1 to the bottom of the tulip-shaped head 3.1.. Due to a direct contact produced by tightening the cylindrical threaded nut 3.2. towards the rod R1, the sacrum plate 1.1. is locked to the rod R1 rigidly and safely. In addition, the implantation rod R1 fixed within the tulip-shaped head 3.1. of the top locking system 3 and extending above the sacrum screws prevents the latter from being loosened. The result is a rigid and safe fixation of the sacrum plate 1.1. to the sacrum bone, on the one hand, and a safe fixation of the implantation rod R1 to the sacrum plate 1.1., on the other hand.

In a similar way, a rigid and tight connection may be established between a monoaxial screw 1.4. and an implantation rod R1. This is shown in Figure 6D. The monoaxial screw 1.4. shown in Figure 6D comprises a (preferably cylinder-shaped) threaded lower end 1.4.3., an unthreaded intermediate part 1.4.2. and a top part 1.4.1. to which the tulip-shaped head 3.1. is connected in one piece, i. e. integrally. Once the monoaxial screw 1.4. is inserted and screwed into a hole drilled into a bone (or is directly screwed into the bone) and received by the bone securely, the tulip-shaped head 3.1. may receive, in its U-shaped indentation 3.1.6., an implantation rod R1 which may be fixed by inserting, and screwing towards the rod R1, a cylindrical threaded nut 3.2.. Also such a system is called a top locking system in accordance with the present invention. On such a way, a rigid connection between the monoaxial screw 1.4. and an implantation rod R1 can be established.

In accordance with the invention, the outer thread of the monoaxial screw 1.4. may be cylindrical (as mentioned above) but may also have any other shape (e. g. conical) known from such screws. This will be shown below when describing the mono-axial screw 1.4. of the system in more detail.

In a very similar way, a monoaxial expansive screw 1.3. as shown in Figure 6E (described below in detail) or a hook 1.9. as shown in Figure 6F (also described below in detail) may be provided with the top locking system 3. In accordance with the invention, also these means allow a rigid and reliable fixation to an implantation rod R1 after inserting, and screwing towards said rod R1, the respective cylindrical threaded nuts 3.2..

In a way making use of the same principles as explained above with respect to the other fixation means, the top locking system may be applied to a polyaxial screw 1.2. of the present invention and explained in detail below. This is shown in Figures 6G, 6H and 6I.

As shown in Figure 6G in the form of a lateral explosion view, the polyaxial screw 1.2. using the top locking system 3. in accordance with the invention may comprise the tulip-shaped head 3.1. having the same structure as already explained above, the polyaxial screw body 1.2.1. comprising the lower threaded end 1.2.5. (having either a cylindrical shape or any other (e. g. conical) shape conceivable for such screws), an intermediate part 1.2.4. without thread and a head 1.2.2. having circumventing indentations 1.2.2. on its lower side, and a cylindrical threaded nut 3.2. which may be inserted into the upper thread 3.1.2. of the tulip-shaped head 3.1. and screwed towards an implantation rod R1 which is inserted into and received by the U-shaped indentations 3.1.6. between the cylindrical threaded nut 3.2. and the upper surface of the polyaxial screw 1.2.. Polyaxiality of the rod fixation is obtained (as is described in detail below) by receiving the rod within the recess 3.3.1. of an intermediate part 3.3. arranged between the tulip-shaped head 3.1. and the cylindrical screw 3.2., the lower hemispherical part 3.3.2. of which is received by the hollow part of the head of the polyaxial screw 1.2. to allow a fixation of the rod along a plurality of conceivable axes ("polyaxial screw"). This will be described in more detail below.

In a particularly preferred embodiment of the invention applied to the practice, the parts shown in Figure 6G are delivered in a state where they are already assembled to a polyaxial screw assembly.

Figure 6H shows the same preferred embodiment of the invention as Figure 6G, wherein the polyaxial screw 1.2. using the top locking system 3. in accordance with the invention comprises, in addition, said intermediate element 3.3. comprising a U-shaped part 3.3.1. adapted to house an implantation rod R1, R2, R3, ... and a hemispherical part 3.3.2. on the lower side of said U-shaped part 3.3.1. adapted to be housed in a corresponding hollow part of the head of the polyaxial screw in a way allowing a movement of the hemispherical part 3.3.2. of the intermediate part 3.3. within the hollow part in the head of the polyaxial screw 1.2. as long as the ensemble is not yet fixed by screwing the cylindrical threaded nut 3.2. into the inner part of the tulip-shaped head 3.1. of the top locking system 3 and adapted to evenly transmit forces received from the U-shaped part 3.3.1. radially as soon as the fixation has been made to the U-shaped part 3.3.2. of the intermediate part 3.2. When screwing the cylindrical threaded nut 3.2. towards the annular base of the tulip-shaped head 3.1., a pressing force is exerted onto the implantation rod R1 housed in the U-shaped indentation 3.1.6. of the tulip-shaped head 3.1. and received by the U-shaped part 3.3.1. of the intermediate element 3.3.. Said pressing force is transmitted evenly in a radial manner to the annular base 3.1.5. of the tulip-shaped head in order to distribute it and prevent that the force be applied to a single location of the head 3.1.. The even distribution of the force can best be seen from the arrow distribution in Figure 6I. This Figure also shows that a large number of orientations of the polyaxial screw 1.2. with respect to the axis of the tulip-shaped head 3.1. housing the implantation rod can be realized, allowing the surgeon much more freedom in fixing the implantation rod to the screws screwed into the bone parts of the respective vertebra.

As can also be derived from Figure 6I, the polyaxial screw 1.2. may be fixed at the bottom of the tulip-shaped head 3.1., i. e. close to the annular base thereof, either at an angle of 0 ° (given as the angle between the axis of the tulip-shaped head 3.1. and the axis of the polyaxial screw body 1.2.) or at an angle different from 0 °, e. g. at an angle of 1 to at least 20 ° to both sides, thus creating a polyaxiality favorable for the system of the present invention in cases where the implantation rod is not placed in a position exactly parallel to the tulip base. This gives the surgeon a better flexibility when fixing the implantation rod by means of the screw system.

Figures 6J to 60 show fixation examples of implantation rods R1, R2, R3, ..., by means of the top locking system 3 of the invention.

In Figure 6J, four monoaxial screws 1.4. received, in the tulip-shaped heads 3.1. of the top-locking system 3 mounted on their top, one implantation rod R1, and the inserted rod R1 was fixed within the tulip-shaped heads 3.1. by means of cylindrically threaded fixation nuts 3.2.. Apparently, this embodiment achieves a safe and rigid fixation of the implantation rod R1 to the spinal column via the monoaxial screws 1.4..

In another preferred embodiment of the invention (without Figure), the embodiment shown in Figure 6J with monoaxial screws 1.4. may be realized with monoaxial expansive screws 1.3., as they are shown in Figures 6E and 9A and 9B. In this case, the safe and rigid fixation can be achieved, too, by extending the lower tip of the screw by screwing in the expansion rod into the inner channel of the screw.

In another preferred embodiment (at least partly also shown in Figure 6J), the monoaxial screws 1.4. (or monoaxial expansive screws 1.3.) may be mounted with washers 3.4. below their tulip-shaped heads 3.1., which washers may bear spikes 3.4.1. allowing an even better fixation of the system to the cortical bone into which the spikes 3.4.1. enter when the screws are screwed in.

Figures 6K, 6L and 6M show perspective, top elevational and side elevational views, respectively, of a connection between two parallel implantation rods R1 and R2 and an open parallel connector 3.5., together with a fixation of the implantation rods R1 and R2 via four top locking system monoaxial screws, two of which are monoaxial screws 1.4. without expansion and two of which are monoaxial expansive screws 1.3.. The use of both monoaxial screws and monoaxial expansive screws together is optional, and the invention may use one type thereof, only, i. e. only four monoaxial screws or only four monoaxial expansive screws, in other embodiments which are also preferred. A skilled person and, in particular, a surgeon, may select the use of these (and other types of) screws in accordance with the needs. Of course, the implantation rods R1 and R2 used in an embodiment of the invention as shown in Figures 6K, 6L and 6M may have identical length and/or identical diameter; however, their lengths and diameters may also be different. The respective two rods are fixed in a strictly parallel position to each other by means of at least one open parallel connector 3.5.. More than one open parallel connector 3.5. may be used, if necessary in a specific case. Apparently, the embodiment exemplified in Figures 6K, 6L and 6M provides a desired stable and safe parallel fixation of implantation rods in cases of anterior surgical approaches.

In particularly preferred embodiments of the invention (and as shown in Figure 6K and 6M for the monoaxial expansive screw 1.3. and the monoaxial screw 1.4. appearing in the Figure on the upper right or right sides, respectively), the screws may be provided with washers containing spikes in order to even more improve the fixation to the bone material.

Figures 6N and 60 show perspective and top elevational views, respectively, of combinations of two implantation rods R1 and R2 having different lengths with top locking system monoaxial screws 1.4. and one open parallel connector 3.5. Of course, for such types of combinations, the number of connectors 3.5., or top locking system monoaxial screws 1.4. and the length of the implantation rods is not restricted to the embodiment of the anterior assembly shown in Figures 6N and 60. The system shown provides an even more stable and safe fixation over an extended length of the spinal column of a human than the system described above.

In addition, in a preferred embodiment of the invention, one or all of the monoaxial screws 1.4. in the system shown may be replaced by monoaxial expansive screws 1.3., if this is considered necessary by a skilled person, e. g. a surgeon. Another preferred embodiment consists in one or two or all of the screws being provided with washers with or without spikes so as to even more improve the fixation of the implantation rods R1 and R2 to the bone material.

In accordance with the invention, the system of means allowing a fixation of at least one implantation rod R1, R2, R3, ... to at least parts of the spinal column of a human comprises, in addition to at least one implantation rod R1, R2, R3, ..., at least one of a number of fixation means selected from a sacrum plate 1.1.; at least one polyaxial screw 1.2.; at least one monoaxial expansive screw 1.3.; at least one monoaxial screw 1.4.; at least one multidirectional coupler 1.5.; at least one multidirectional offset coupler 1.6.; at least one side loading expansive screw 1.7.; at least one transverse connector 1.8.; at least one hook 1.9.; at least one closed parallel connector 1.10.; at least one coaxial connector 1.11.; at least one multidirectional double coupler 1.12.; and at least one injection screw 1.13..

The above means may be used as one means alone with at least one implantation rod R1, R2, R3, ... without restriction. It is, however, possible and is a preferred embodiment of the invention that two or more of the means mentioned above and described in this description in detail be used in combination, whereby great advantages of each of the means contribute to an outstanding performance of the whole system used. If two or more means are used, they may be identical or may be different and may be selected from the above means without any restriction and only subject to the specific anatomic situation. Particularly, it is possible, by combining two or more of the above means, to give the surgeon, in the course of a surgery, all freedom to apply those means alone or in combination which he considers appropriate in a specific situation and under specific requirements of the constitution of a specific human to be subjected to surgery.

One of the embodiments of the system according to the invention employs, in combination with other means, a sacrum plate 1.1.. The sacrum plate 1.1. has the purpose of achieving a fixation of at least one implantation rod R1, R2, R3, ... in the area of the sacrum bone. The sacrum plate, which is used with two sacrum screws to achieve a highly secured anchorage to the bone since, at this level, the forces applied to the implantation rod are higher than at other spine levels, is fixed to the sacrum bone by two specific sacrum screws which are guided through two channels the angles of which, relative to the plane of the sacrum plate are not parallel and are not both 90 °, but are different by three angles (α, β and y which are referred to as the transversal plane angle (not parallel in a view parallel to the plane of the sacrum plate), the sagital plane angle (not parallel in a view along the plane of the sacrum plate) and the front plane angle (not parallel in a view from the top of the sacrum plate). The polyaxiality of the two sacrum screws achieving a fixation of the sacrum plate to the sacrum bone allows an orientation of the screw to those areas where there can be found the maximum cortical bone in the sacrum. It is particularly advantageous that the sacrum screws used for a fixation of the sacrum plate to the bone are self-locked against pull-out from the plate. This is achieved by the fact that, once both sacrum screws are screwed in and the implantation rod has been locked in the tulip head of the top locking system by means of the cylindrical threaded nut, the position of the implantation rod remains just above the heads of the sacrum screws, thus avoiding that they may come out from its relative position within the sacrum plate.

In a particularly preferred embodiment of the invention, the sacrum plate 1.1. is provided with a top locking system 3 which is explained in detail hereinabove. In a further preferred embodiment, the top locking system 3 is provided on that side of the sacrum plate from which the sacrum screws are inserted (which is the side opposite to the side where the threaded screws are fixed to the sacrum bone).

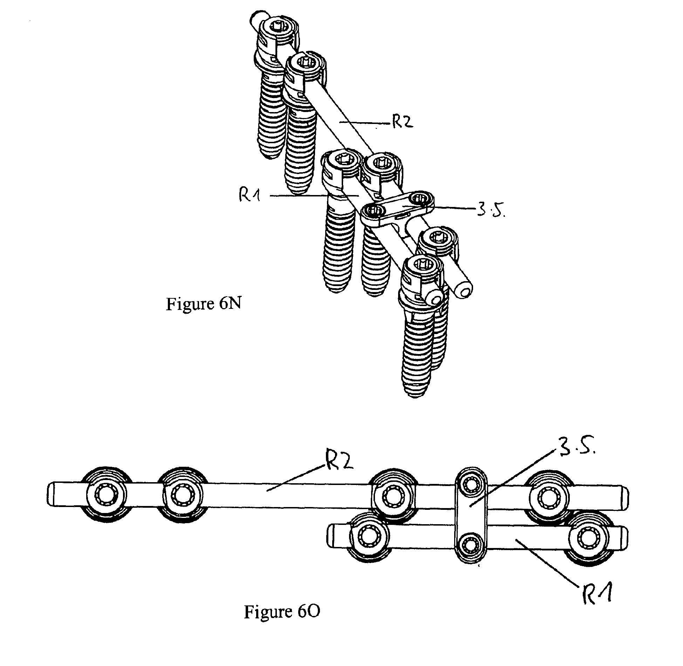

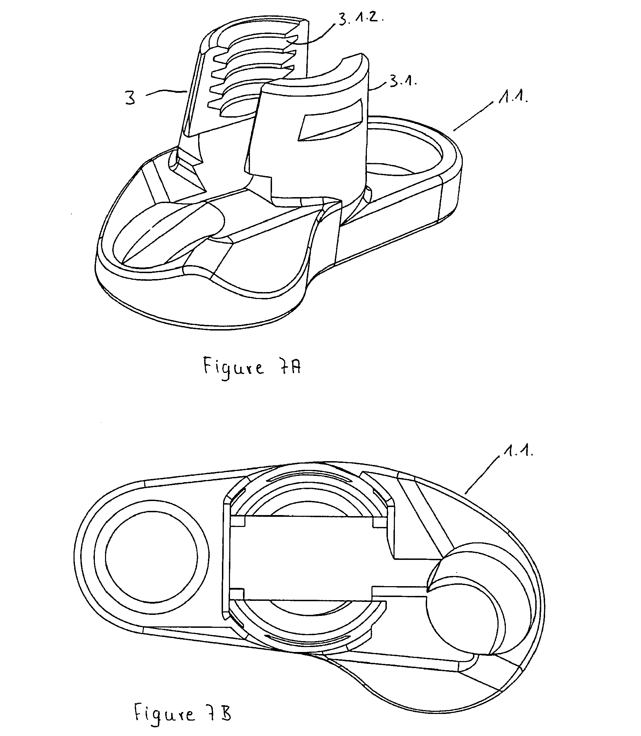

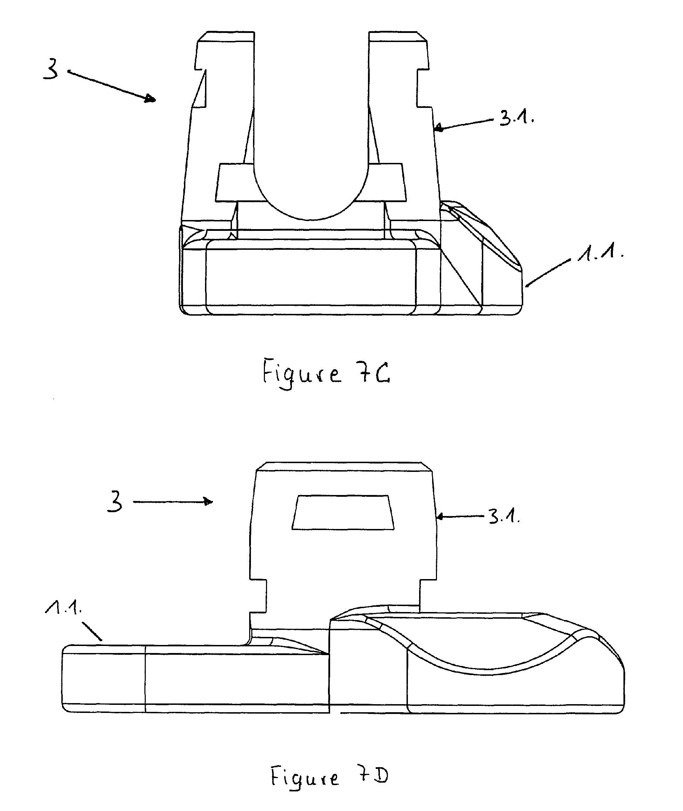

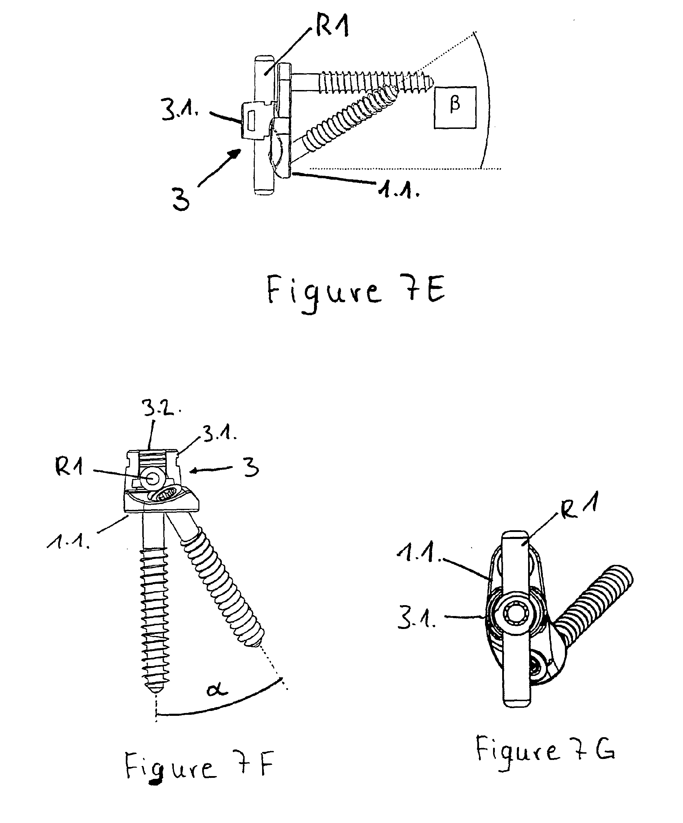

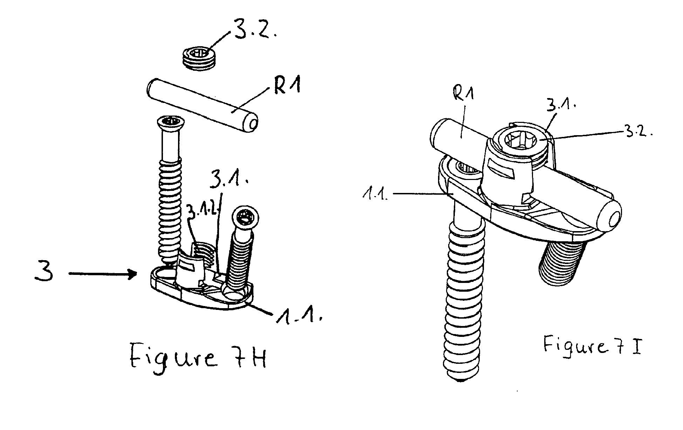

The sacrum plate 1.1. including the top locking system is exemplified by the Figures 7A to 71, wherein Figures 7A to 7D show perspective, side, front and top views of the sacrum plate 1.1., Figures 7E to 7G show the angle characteristics of the sacrum plate of the invention, Figure 71 shows the complete arrangement of the sacrum plate assembly 1.1. in a mounted stage (i. e. mounted by screwing in the sacrum screws into the bone and by fixing the implantation rod to the top lock system (described below) of the sacrum plate by means of a fixation nut). Figure 7H shows an explosion view showing in detail all parts of the sacrum plate system 1.1. before mounting.

In another preferred embodiment, the system of the invention comprises at least one polyaxial screw 1.2. alone or in combination with other means as mentioned above for fixing an implantation rod R1, R2, R3, .... In an even more preferred embodiment, the system comprises at least one polyaxial screw 1.2. which is provided with a top locking system 3.. The details of this preferred embodiment are described above, and a repetition is not necessary here. There may be used one polyaxial screw 1.2., or two, three, four, or any desirable number of polyaxial screws 1.2. may be used.

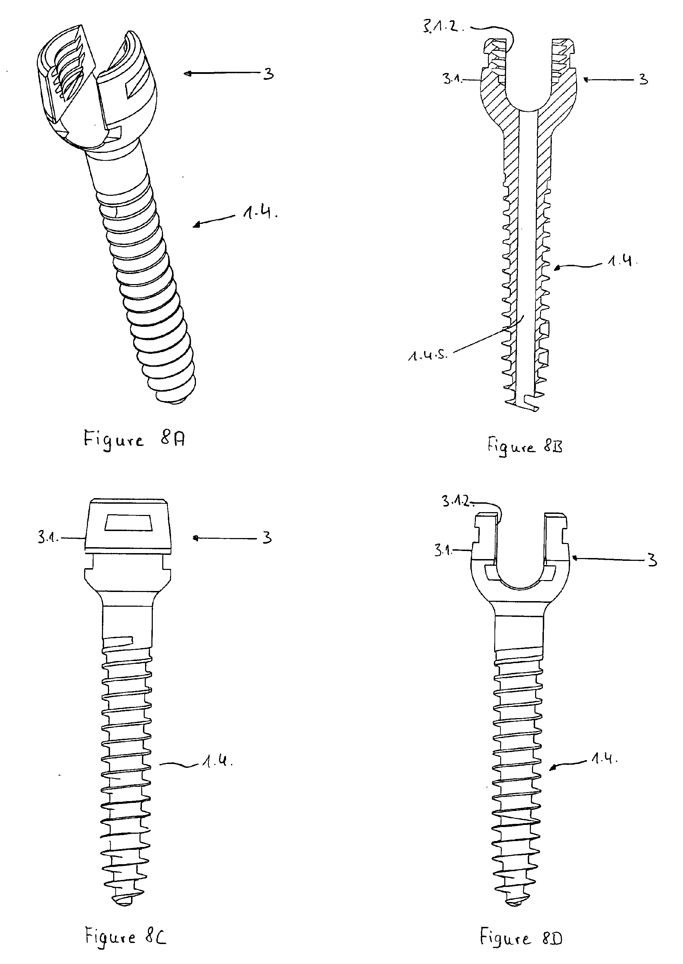

A further preferred embodiment of the invention relates to a system of the invention comprising at least one monoaxial screw 1.4. alone or in combination with other means as mentioned above for fixing an implantation rod R1, R2, R3, .... In an even more preferred embodiment, a monoaxial expansive screw 1.3. is provided with a top locking system 3 as mentioned above. Details of this preferred embodiment are described above, and a repetition is not necessary here. This particularly preferred embodiment of the invention is shown in Figures 8A to 8D. The perspective view of Figure 8A shows a cylindrical shape of the lower threaded part of the monoaxial screw 1.4. of the invention, while the other Figures 8B to 8D show other shapes of the threaded part of the screw. Even a cannulated version of said monoaxial screw 1.4. as shown in Figure 8B may be made, wherein the channel has the reference numeral 1.4.5. and is extending from the tulip-shaped head 3.1. to the tip of the screw 1.4.3.. The cannulated moniaxial screw comprises the threaded shaft on top of which the tulip-shaped head 3.1. is mounted. From the bottom of the tulip-shaped head through the inner part of the shaft of the screw, a channel is drilled which extends down to the bottom tip 1.4.3. thereof for receiving a wire guide or navigation wires allowing the position of the screw to be determined by systems (per se known in this field of the art) like X-ray or ultrasound. This embodiment has the advantage that it can be helpful for introducing a wire through the pedicle for guidance purposes before introducing the pedicular screw, preferably in examples of minimal invasive surgery. There may be used one monoaxial screw 1.4., or two, three, four or any desirable number of monoaxial screws 1.4. may be used.

A further preferred embodiment of the invention relates to a system of the invention comprising at least one monoaxial expansive screw 1.3. alone or in combination with other means as mentioned above for fixing an implantation rod R1, R2, R3, .... In an even more preferred embodiment, a monoaxial expansive screw 1.3. is provided with a top locking system 3 as mentioned above. Details of this preferred embodiment are described above, and a repetition is not necessary here. The monoaxial expansive screw 1.3. is shown in Figures 9A to 9C in detail as a preferred embodiment, which Figures show three-dimensional perspective explosion, three-dimensional perspective mounted and sectional views, respectively. Basically, the monoaxial expansive screw comprises the threaded screw body 1.3.3. as the lower part, which may be cylindrical in shape, but also may have any other shape known to a skilled person in this technical field. For example conical shapes are possible, too. The screw 1.3. may comprise an intermediate, optionally unthreaded part 1.3.2 and comprises a top part or head 1.3.1. which may take the shape of a tulip-shaped head 3.1. in case of the embodiment including the top-locking system 3.

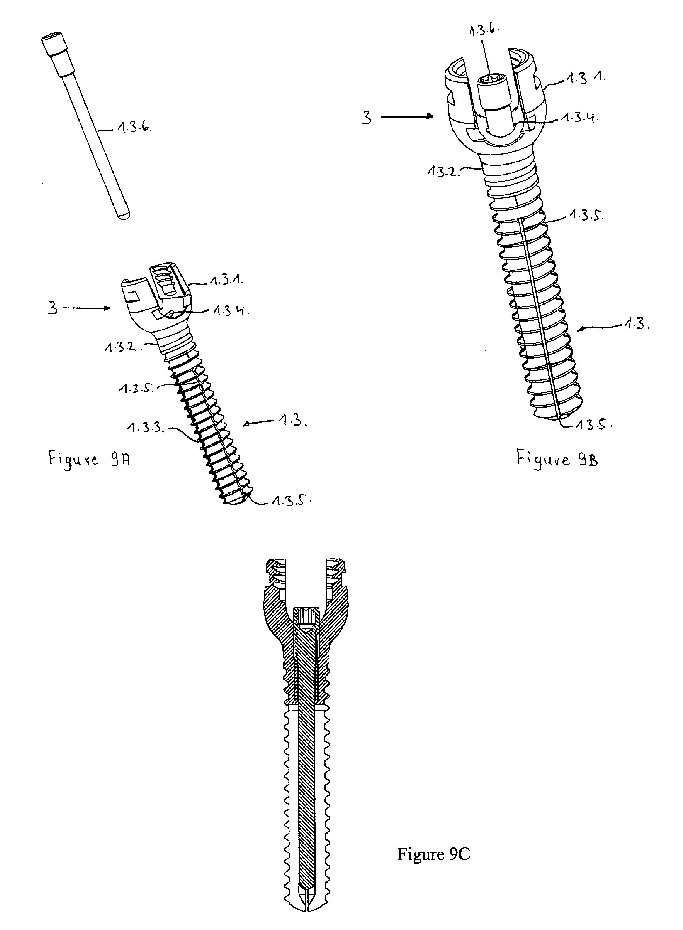

In order to allow the threaded screw body to be expanded, several (per se known) designs of the screw body may be provided which are suitable for the intended purposes. In a particularly preferred embodiment shown in Figure 9A, the screw body 1.3.3. has an inner channel 1.3.4. and, at the lower part close to the lower tip, even more preferred from, for example, about half the total length of the screw body 1.3.3. to the lower tip, has one or more than one, preferably two, three or four, slit(s) 1.3.5. extending from the inner channel 1.3.4. to the outer surface of the screw body 1.3.3. allowing an extension thereof. The extension of the screw tip is effected by inserting, into the inner channel, a plug 1.3.6., or by screwing into the inner channel (having a thread at its inner walls) a screw 1.3.6., said plug or screw 1.3.6. having a diameter larger than the inner diameter of the channel 1.3.4. or increasing in diameter beyond the inner diameter of the channel at a specific position, as shown in Figure 9B. Alternatively, said plug or screw 1.3.6. may be constructed in a way having a constant diameter along its length, while the inner diameter of the channel is decreased along its length continuously or discontinuously so as to be extended by inserting, or screwing in, the constant diameter plug or screw 1.3.6.. The extended monoaxial expansive screw 1.3. may achieve an even better fixation of the screw body 1.3.3. to the bone and, as a consequence, may also improve the fixation of the implantation rod to the spinal column via the monoaxial expansive screw 1.3.. Figure 9C shows a side sectional view of the monoaxial expansive screw 1.3.. As will be recognized, the inner channel 1.3.4. extends through the screw body 1.3.3. from the annular base of the tulip-shaped head 3.1. to the lower tip of the screw and is suitable to receive the plug 1.3.6. or screw 1.3.6..

Particularly in a case of a top locking system provided to the top of the monoaxial expansive screw 1.3., as shown in Figures 9A to 9C, a reliable and rigid fixation to the implantation rod(s) R1, R2, R3, ... can be achieved.

There may be used one monoaxial expansive screw 1.3., or two, three, four or any desirable number of monoaxial expansive screws 1.3. may be used, of which none, one, two, ..., or all of them may be provided with the top locking system 3 including the tulip-shaped head 3.1..

A further preferred embodiment of the invention relates to a system of the invention comprising at least one multidirectional coupler 1.5. alone or in combination with other means as mentioned above for fixing an implantation rod R1, R2, R3, .... There may be used one multidirectional coupler 1.5., or two, three, four or any desirable number of multidirectional couplers 1.5. may be used.

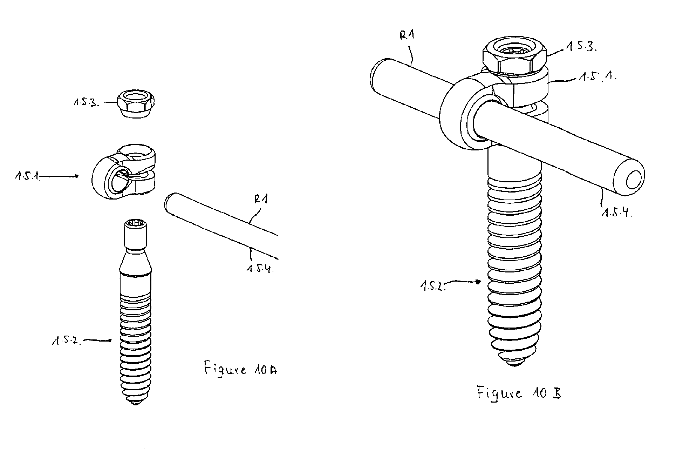

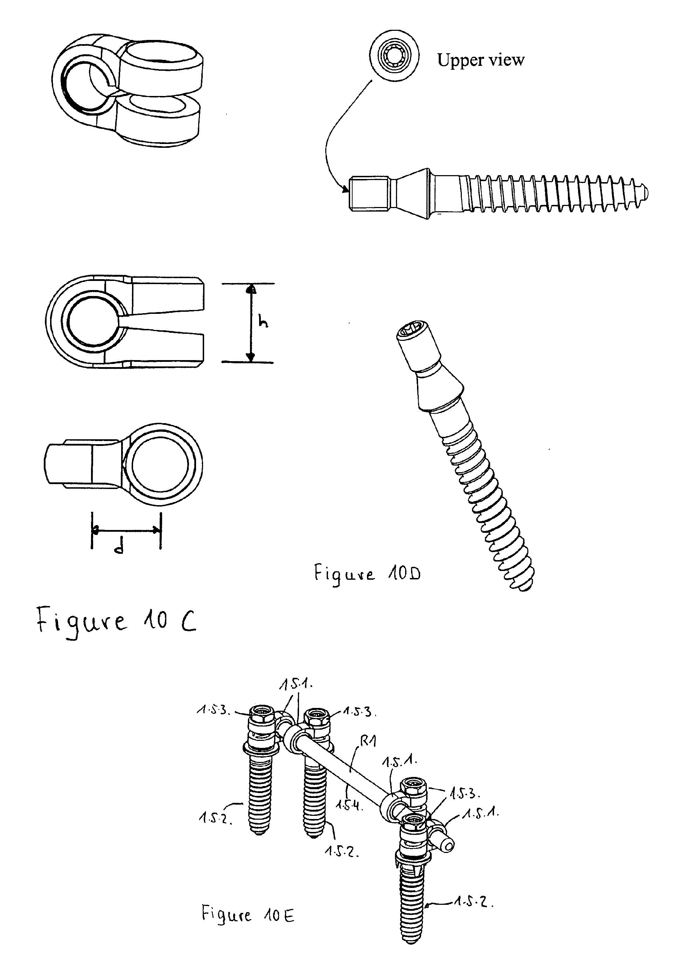

In accordance with this preferred embodiment of the invention and as shown in Figure 10A as an explosion view, the multidirectional coupler 1.5. comprises a screw component 1.5.2. having a cylindrical or non-cylindrical (e. g. conical) threaded screw part which is limited at its upper end by a radially widened portion preventing that the screw component 1.5.2. is inserted too far into any hole drilled into the bone or is screwed too far directly into the bone for achieving a fixation. In contrast to prior art screw components, said radially widened portion does not comprise a hexagon for operation of the screw component 1.5.2., but is designed as a conical portion having said radially widened portion as a base.

Said conical portion is designed to accommodate the multidirectional coupler component 1.5.1.. In an axial direction towards the top of the screw component 1.5.2. and above the conical portion, there is arranged a cylindrical portion of the screw component 1.5.2.. At the top of the screw component 1.5.2., there is provided a threaded cylindrical portion for receiving the nut 1.5.3. fixing the multidirectional coupler component 1.5.1. to the screw component 1.5.2. and - simultaneously - fixing the multidirectional coupler component 1.5.1. to the implantation rod R1, R2, R3, ... through the internal sphere of the multidirectional coupler. In addition, the top of the screw component 1.5.2. received a hexalobe allowing on operation of the screw component 1.5.2. by means of a suitable tool. Figure 10B shows the multidirectional coupler 1.5. completely mounted and including the rod 1.5.4..

In a comparison shown in Figure 10C, the multidirectional coupler 1.5. of the present invention is shown. Compared to one multidirectional coupler design of the prior art, in the multidirectional coupler component 1.5.1. of the present invention, a lower profile could be obtained by reducing the diameter d of the sphere being fixed to the implantation rod and by shortening the distance between the centers of the circles/spheres serving the fixation of the screw body and of the implantation rod (Figure 10C). The diameter reduction may be, for example, 1 mm, without being restricted to said value, as also the distance reduction may be 1 mm, without being restricted to said value. In Figure 10 D, the screw 1.5.2. of the present system is shown. It is compared to the screws of the prior art. For the screws of the invention, a lower profile could be obtained by omitting the hexagon at the radially widened portion (this was replaced by a hexalobe in the top portion of the screw) and shortening the distance between the radially widened portion and the top of the screw.

The multidirectional coupler component explained above may, for example, be used for anterior assemblies of implantation rods as exemplified by the Figures 10E to 10I. Figures 10E to 10G show perspective, side elevational and top elevational views, respectively, of single rod constructs where one implantation rod R1 is fixed by the multidirectional coupler components 1.5.1. of four screw components 1.5.2.. Of course, the number of four screw components is not limiting, and there may be fewer or more, as needed. Particularly, the screw components need not necessarily to be arranged in pairs or at the respective ends of the implantation rod R1, but may be located along the length of the implantation rod R1 as needed in connection with the respective application. A particularly reliable fixation of the implantation rod may be obtained by the construction shown in Figures 10E to 10G.

According to another preferred embodiment of the invention, one or two or all of the screws are provided with washers with or without spikes so as to even more improve the fixation of the implantation rod R1 to the bone material. This is, for example, shown in Figure 10E for the screw component being located in the "foreground" of the perspective view of this embodiment.

Top perspective and top elevational views of another preferred embodiment of the invention, wherein, in a double rod construct for an anterior assembly, two implantation rods R1 and R2 are fixed to the spinal column of a human, are shown in Figures 10H and 10I. While the fixation of the independent rods R1 and R2, respectively, is afforded in the same way as already described in connection with Figures 10E to 10G, i. e. by connecting the respective rod R1 or R2, respectively, to the multidirectional couplers 1.5.1. of two or three screw components 1.5.2., the rods are fixed in a position essentially parallel to each other by means of a transverse connector, thus allowing a reliable and rigid fixation of two implantation rods.

A further preferred embodiment of the invention relates to a system of the invention comprising at least one multidirectional offset coupler 1.6. alone or in combination with other means as mentioned above for fixing an implantation rod R1, R2, R3, .... There may be used one multidirectional offset coupler 1.6., or two, three, four or any desirable number of multidirectional offset couplers 1.6. may be used.

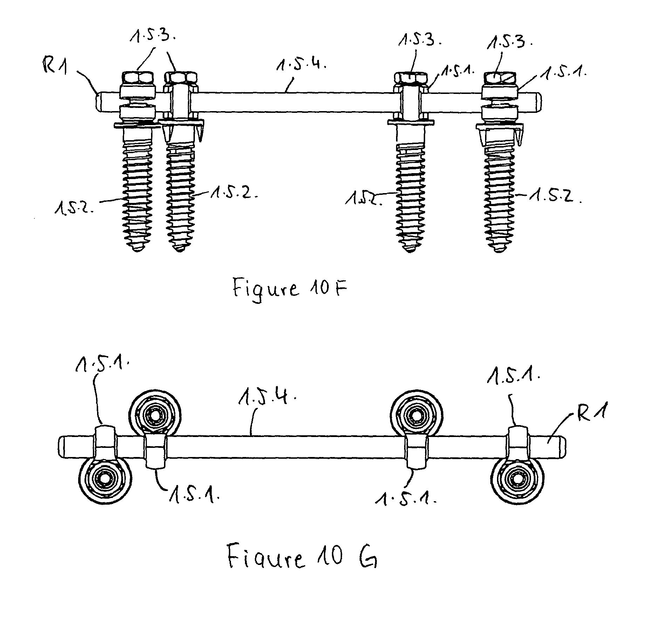

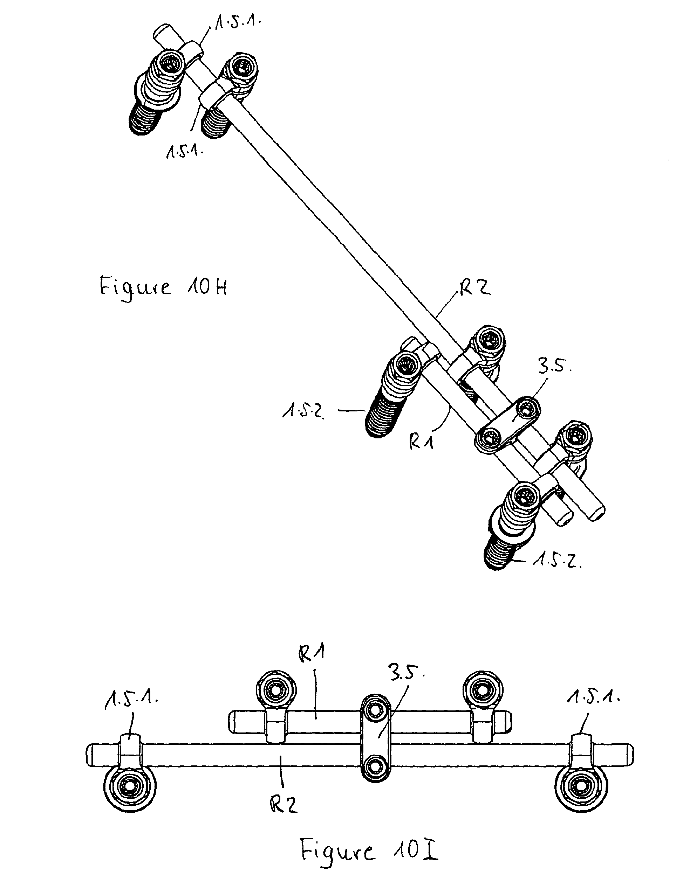

In accordance with this preferred embodiment of the invention and as shown in Figure 11A, the multidirectional offset coupler 1.6. comprises a screw component 1.6.2. having a cylindrical or non-cylindrical threaded screw part which is limited at its upper end by a radially widened portion preventing that the screw component 1.6.2. is inserted too far into any hole drilled into the bone for achieving a fixation. In contrast to prior art screw components, said radially widened portion does not comprise a hexagon for operation of the screw component 1.6.2., but is designed as a conical portion having said radially widened portion as a base and being designed to accommodate the multidirectional offset coupler component 1.6.1.. In an axial direction towards the top of the screw component 1.6.2. and above the conical portion, there is arranged a cylindrical portion of the screw component 1.6.2.. At the top of the screw component 1.6.2., there is provided a threaded cylindrical portion for receiving the nut 1.6.3. fixing the multidirectional offset coupler component 1.6.1. to the screw component 1.6.2. and - simultaneously - fixing the multidirectional coupler component 1.6.1. to the implantation rod R1, R2, R3, ... through the internal sphere of the multidirectional coupler. In addition, the top of the screw component 1.6.2. received a hexalobe allowing on operation of the screw component 1.6.2. by means of a suitable tool. Figures 11 B and 11C show the multidirectional offset coupler 1.6. completely mounted and including the rod 1.6.4. as three-dimensional perspective end elevational side views, respectively.

In Figure 11 D, the multidirectional offset coupler 1.6. of the present invention is shown. In comparison to one multidirectional offset coupler design of the prior art, the multidirectional offset coupler component 1.6.1. of the present invention as shown in Figure 11D exhibits a lower profile having a reduced diameter of the sphere while being fixed to the implantation rod. By this embodiment, the distance between the centers of the circles/spheres serving the fixation of the screw body and of the implantation rod (Figure 11 D) is shortened. The diameter reduction may be, for example, 1 mm, without being restricted to said value, as also the distance reduction may be 1 mm, without being restricted to said value. In addition, the screws of the present system are shown to be different from those of the prior art. For the screws in the multidirectional offset coupler component of the embodiment of the invention, a lower profile could be obtained by omitting the hexagon at the radially widened portion (this was replaced by a hexalobe in the top portion of the screw) and shortening the distance between the radially widened portion and the top of the screw.

A further preferred embodiment of the invention relates to a system of the invention comprising at least one side loading expansive screw 1.7. alone or in combination with other means as mentioned above for fixing an implantation rod R1, R2, R3, .... There may be used one side loading expansive screw 1.7., or two, three, four or any desirable number of side loading expansive screws 1.7. may be used.

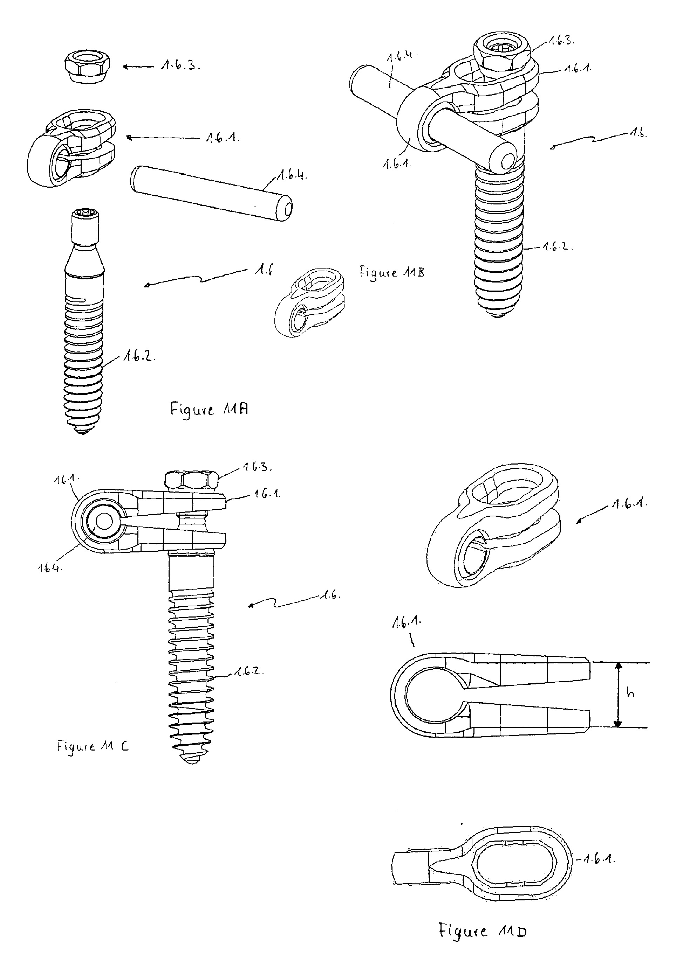

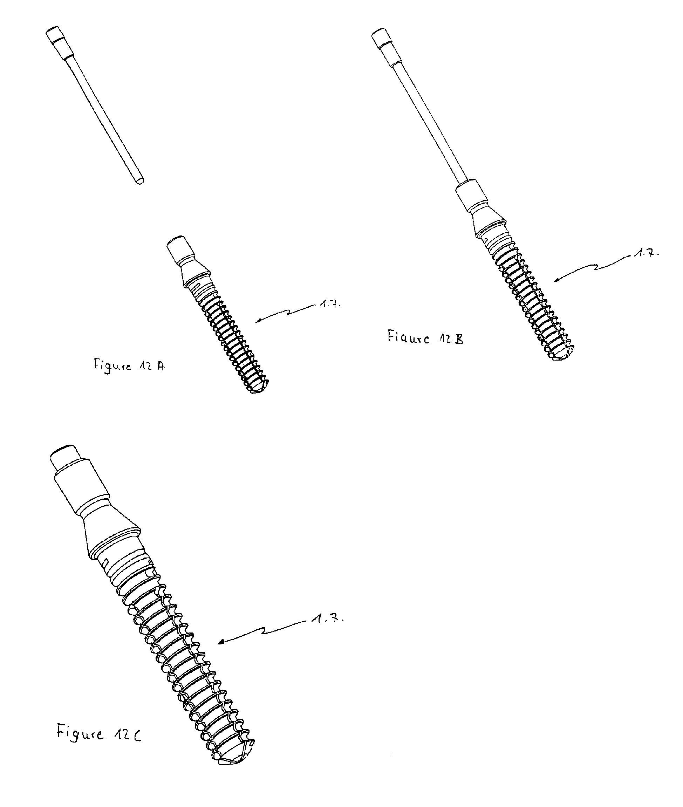

The side loading expansive screw used in accordance with the present invention alone or in combination with the other system means as described is shown in Figures 12A to 12C. It is distinguished from the monoaxial expansive screw 1.3. of the present invention by the omission of the tulip head allowing the top lock fixation of the implantation rod. Beyond that, with respect to a description of the structure of the expansive screw and its functions, reference may be made to the description of the monoaxial expansion screw 1.3. described above; the explanations apply here *mutatis mutandis.*

A further preferred embodiment of the invention relates to a system of the invention comprising at least one transverse connector 1.8. alone or in combination with other means as mentioned above for fixing an implantation rod R1, R2, R3, .... There may be used one transverse connector 1.8., or two, three, four or any desirable number of transverse connectors 1.8. may be used. The structure and function of the transverse connector 1.8. was described above in all details with reference to Figures 4A to 4D and 5A to 5F, and a repetition of said description is not needed here.

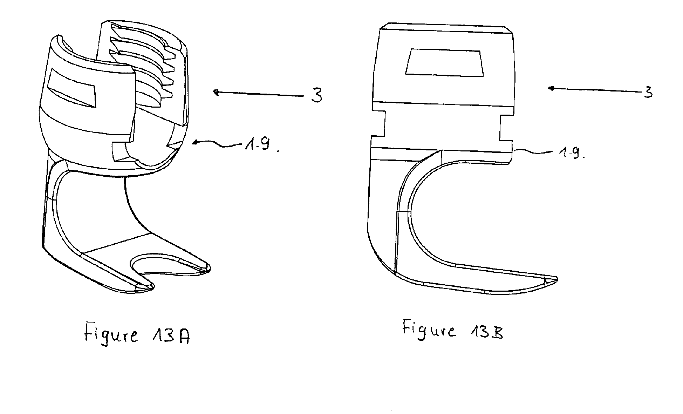

A further preferred embodiment of the invention relates to a system of the invention comprising at least one hook 1.9. alone or in combination with other means as mentioned above for fixing an implantation rod R1, R2, R3, .... There may be used one hook 1.9., or two, three, four or any desirable number of hooks 1.9. may be used. The structure of the hook 1.9. is shown in Figure 13, wherein Figure 13A shows a hook according to one embodiment of the invention as a three-dimensional perspective view, while Figure 13B shows an elevational side view of a hook. The hook may serve for fixing an implantation rod R1, R2, R3, ..., to the hook instead fixing it to a pedicular screw by means of the top locking system 3 using the tulip head 3.1. and its corresponding locking screw. In such a case, the hook is secured to the vertebra by means of its blade, i. e. either to the vertebral lamina or to the pedicle; the latter vertebra fixation is per se known from the prior art, e. g. from the document EP-A 1 254 639. Not known from said document is the top locking system 3 as a means of fixation to the implantation rod.

A further preferred embodiment of the invention relates to a system of the invention comprising at least one closed parallel connector 1.10. alone or in combination with other means as mentioned above for fixing an implantation rod R1, R2, R3, .... There may be used one closed parallel connector 1.10., or two, three, four or any desirable number of closed parallel connectors 1.10. may be used. The structure and function of the closed parallel connector 1.10. was described above in all details by referring to Figures 3A to 3D, and a repetition of said description is not needed here.

A further preferred embodiment of the invention relates to a system of the invention comprising at least one coaxial connector 1.11. alone or in combination with other means as mentioned above for fixing an implantation rod R1, R2, R3, ... . There may be used one coaxial connector 1.11., or two, three, four or any desirable number of coaxial connectors 1.11. may be used. The structure and function of the coaxial connectors 1.11. was described above in all details with reference to Figures 2A to 2C, and a repetition of said description is not needed here.

A further preferred embodiment of the invention relates to a system of the invention comprising at least one multidirectional double coupler 1.12. alone or in combination with other means as mentioned above for fixing an implantation rod R1, R2, R3, .... There may be used one multidirectional double coupler 1.12., or two, three, four or any desirable number of multidirectional double coupler 1.12. may be used. The structure and function of the multidirectional double coupler 1.12. is now described in detail.

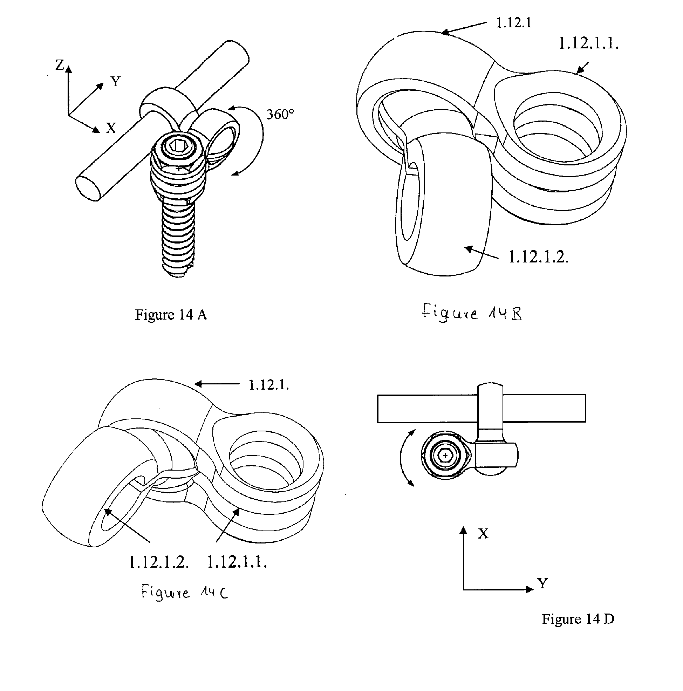

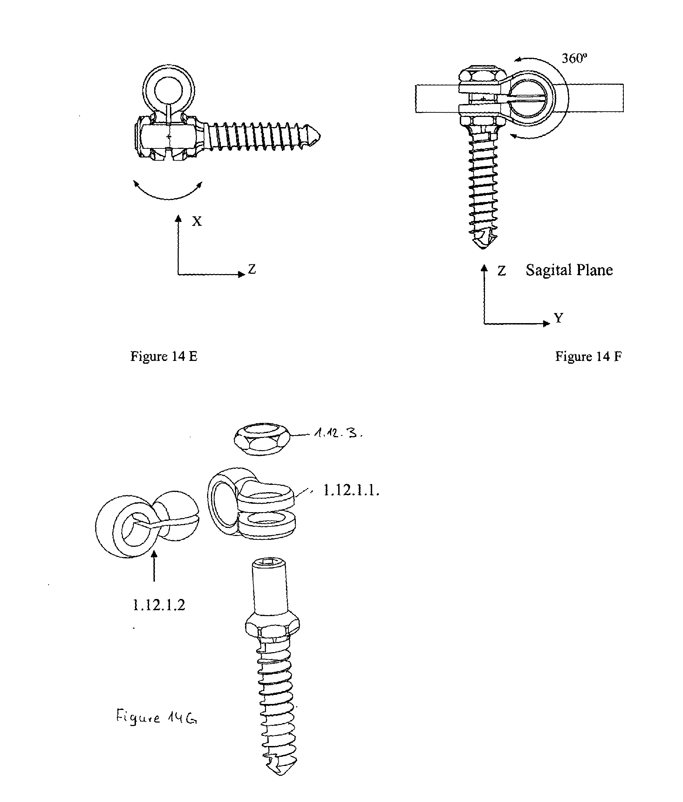

Figures 14A to 14G show the multidirectional double coupler system 1.12. in accordance with the preferred embodiment of the invention. As seen in Figure 14A, the multidirectional double coupler system 1.12. comprises the multidirectional double coupler 1.12.1., which is in more detail shown also in Figures 14B and 14C, the screw 1.12.2. to which the coupler is fixed and which - as described in a similar way above - consists of a lower threaded screw part, an intermediate part optionally provided with a radially widened portion preventing a too deep insertion thereof into a hole drilled in the bone, and a top portion having a thread for the nut 1.12.3.. Further components of the multidirectional double coupler system 1.12. are the nut 1.12.3. for a fixation of the coupler component to the head of the screw and an implantation rod. Figures 14B and 14C show that the multidirectional double coupler 1.12.1. itself is composed of two parts (1.12.1.1., 1.12.1.2.) which are engaged to each other through a joint which may be any suitable joint and usually is a ball joint. Basically, the joint consists of a hollow sphere inside of which another hollow sphere is placed.

Such a construction allows a rotation around three axes (Figures 14D to 14F) and a translation along the rod.

It is particularly advantageous for this embodiment of the invention that only one tightening device is needed to lock the rod to the multidirectional double coupler 1.12.1.: Upon tightening the nut 1.12.3. on top of the screw, one part (1.12.1.1.; see Figures 14B, 14C) is forced to experience a deformation, which, in turn, is transmitted through the sphere onto the other part (1.12.1.2.), which also experiences a deformation and constrains the rod due to the two hemispheres coming closer together.

By the construction of the multidirectional double coupler 1.12., a large degree of freedom of rotation is achieved. This can be seen particularly from Figures 14D to 14F, where all possible axes of rotation of the multidirectional double coupler of the invention are shown separately. Double couplers allow higher angulations than simple couplers in the sagital (or lateral) plane for pedicular, transfacetary or lateral mass screw insertion in high thoratic and cervical spine levels. In particular, free turning around 360 ° in the sagital plane is possible, giving the surgeon more freedom in surgery.

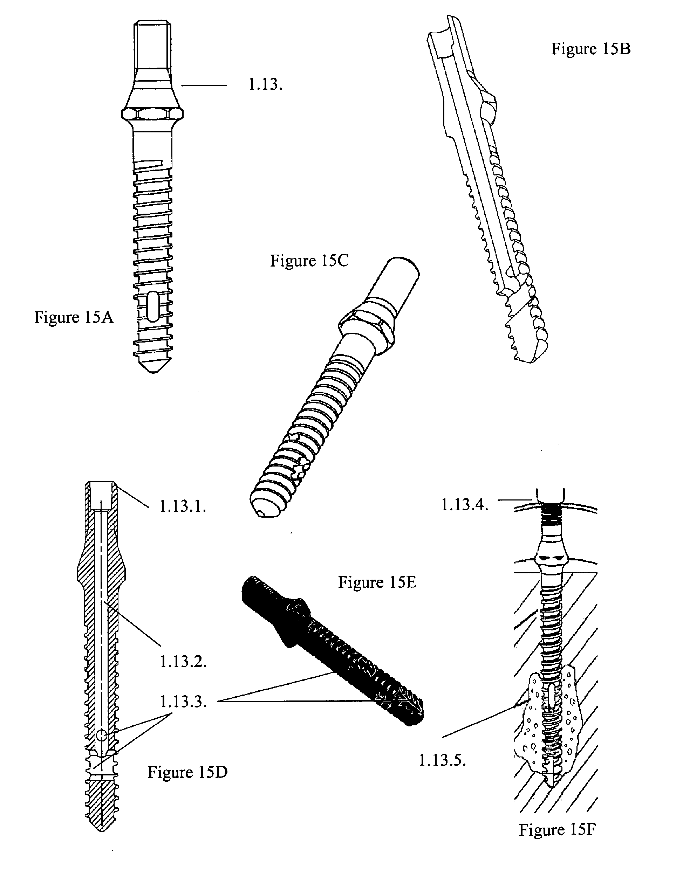

A further preferred embodiment of the invention relates to a system of the invention comprising at least one injection screw 1.13. alone or in combination with other means as mentioned above for fixing an implantation rod R1, R2, R3, ... . There may be used one injection screw 1.13., or two, three, four or any desirable number of injection screws 1.13. may be used. The structure and function of the injection screw 1.13. is now described in detail by means of Figures 15A to 15F.

As shown in Figure 15A as a side elevational view, the injection screw 1.13. may have a similar outer structure as a side loading screw. However, the injection screw 1.13. has an inner main channel or hollow core 1.13.2. (better conceivable from cut away sectional view 15B or sectional view 15D) which extends from an opening 1.13.1. at the top of the injection screw 1.13. in parallel to its axis down to an area of the screw where lateral windows or holes 1.13.3. are found; in any case, the inner main channel 1.13.2. does not extend down to the lower tip of the injection screw 1.13.. In the lower part of the screw, the main channel 1.13.2. opens to one or more openings 1.13.3. which may be windows or holes or slots, preferably 2 to 4 openings 1.13.3., which openings, however, do not reach the lower tip of the screw. In a preferred embodiment of the invention, the openings 1.13.3. are in the form of rectangular windows and/or circular holes and are arranged in such a way that they open from the inner main channel 1.13.2. to the outside environment of the injection screw 1.13. in angles (relative to each other) which may be calculated by [360 ° : (number of openings)], resulting into, for example, 4 openings being arranged in angles of 90 °, relative to each other. It is not compulsory that all openings are located at the same level, i. e. distance, seen from the top of the injection screw; quite to the contrary, it is preferred that the openings or windows 1.13.3. are arranged at different levels. In the embodiments shown exemplarily in Figures 15B, 15C, 15D and 15E, there is one first set of openings 1.13.3. connected by a through channel extending perpendicularly to the inner main channel 1.13.2. which extends from the top of the screw to the openings or windows 1.13.3., said two first openings being arranged in an angle of 180 ° relative to each other, while another pair of openings, e. g. windows or holes ("second openings"), is extending from the inner main channel 1.13.2. to the outside environment 1.13.5. of the screw 1.13., said second openings being arranged in an angle of 180 ° relative to each other but in angles of 90 ° relative to the first openings.

As may be seen from Figure 15F, such an injection screw 1.13. is adapted for bone cement filled into the opening at the top 1.13.1. of the inner main channel 1.13.2. under pressure flowing down the inner main channel 1.13.2. and exiting the inner main channel or hollow core laterally through any of the number of the first and/or second openings 1.13.3. to fill the volume of the intervertebral disc.

Liquid bone cement (or any equivalent like, for example, bone cement substitution materials, growing factor materials etc.) is filled into the channel from a means 1.13.4. fixed on the top 1.13.1. of the screw 1.13., flows down (or is pressed down) the channel 1.13.2. and exits through the lateral openings 1.13.3. into the space 1.13.5. surrounding the injection screw 1.13.. Thereby the cement or its equivalent is evenly distributed around the screw at the area marked with 1.13.5. (as best shown in Figure 15F), thus allowing an improvement of the fixation and anchorage of the screw within a bone, even if it is porous or poor quality bone. The latter situation can be seen best from Figure 15 F. Figures 15C and 15E show three-dimensional perspective views of the injection screw 1.13. of the invention.

All devices described above in detail may be made of any biocompatible material providing the necessary mechanical strength and known to a skilled person from the prior art. Particularly, the present invention contemplates presently (without restriction) making all devices of one single material, as for example stainless steel or titanium alloys; all other biocompatible materials are suitable as well. In addition, it may be advantageous to provide a treatment to the surface of all those parts, particularly to those surfaces which serve the mutual fixation of parts to each other, by which a rough and securely adhering state may be achieved. Measures suitable for this purpose are known to a person skilled in this field and may, for example, comprise a roughening treatment, sandblasting treatment etc.. The latter one may also achieve a surface condition of the parts which the bone growth to the fixation means which promotes a durable and reliable fixation thereof.

The devices of the present invention may be used for immobilizing at least parts of the spinal column, particularly and preferably in a human. Specifically, the devices, alone or in any desirable combination, may be used in spinal surgery, particularly in cases of spinal fusion surgery and/or spinal fixation surgery. In particular, the means of the present invention are useable for the spinal approaches anterior cervical, thoratic and lumbar levels and posterior cervical, thoratic, lumbar and sacrum levels. In the more preferred embodiments of the invention including the injection screws 1.13., these are applicable particularly in cases of
- osteoporosis;
- osteolytic neoplasm (metastasis or primary);
- revision surgery (for example in cases of screw loosening);
- rheumatologic diseases accompanied by a poor bone quality.

The invention finally also relates to a method of fixing at least one implantation rod R1, R2, R3, ... to at least parts of the spinal column of a human, said method comprising the steps of
- selecting at least one implantation rod R1, R2, R3, ... suitable in diameter(s) and length(s) for the intended implantation;
- either drilling at least one hole into at least two vertebrae, or preparing the space around the laminae or vertebral pedicle, at locations allowing a fixation of said at least one implantation rod to the spinal column at a desired position;
- inserting at least one, preferably at least two, of the following means:
   ■ at least one sacrum plate 1.1.;
   ■ at least one polyaxial screw 1.2.;
   ■ at least one monoaxial expansive screw 1.3.;
   ■ at least one monoaxial screw 1.4.;
   ■ at least one side loading screw 1.5.2.;
   ■ at least one side loading expansive screw 1.7.;
   ■ at least one injection screw 1.13.;
- into the hole(s) drilled
- and/or fixing to the space prepared around the laminae or vertebral pedicle
   ■ at least one hook 1.9.
- surrounding said pedicle or lamina; and
- fixing to any one or more of the side loading screws 1.5.2. or side loading expansive screw 1.7. or injection screw 1.13. any other suitable screw at least one of
   ■ at least one multidirectional coupler 1.5.;
   ■ at least one multidirectional offset coupler 1.6.;
   ■ at least one multidirectional double coupler 1.12.;
- so as to allow a fixation of said at least one implantation rod R1, R2, R3, ... to said spinal column; and
- optionally further stabilize the arrangement of the above means by the application, between at least two of said implantation rods R1, R2, R3, ..., of at least one of
   ■ at least one closed parallel connector 1.10.;
   ■ at least one coaxial connector 1.11.;
   ■ at least one transverse connector 1.8..

The invention was described above in connection with its preferred embodiments, which are, however, described only as non-restricting examples of the invention. A skilled person, when reading the description of the preferred embodiments, will be able to derive from these preferred embodiments suitable modifications and equivalents of the invention. The scope of the invention is not restricted by the above description of the preferred embodiments, but can be seen best from the claims which follow.

## Claims

1. A system of means allowing a posterior or anterior fixation of at least one implantation rod (R1, R2, R3, ...) to at least parts of the spinal column of a human, comprising at least one implantation rod (R1, R2, R3, ...) and at least one, preferably at least two of the following means:
- a sacrum plate (1.1.);
- at least one polyaxial screw (1.2.);
- at least one monoaxial expansive screw (1.3.);
- at least one monoaxial screw (1.4.)
- at least one multidirectional coupler (1.5.);
- at least one multidirectional offset coupler (1.6.);
- at least one side loading expansive screw (1.7.);
- at least one set of transverse connectors (1.8.);
- at least one hook (1.9.);
- at least one closed parallel connector (1.10.);
- at least one coaxial connector (1.11.);
- at least one multidirectional double coupler (1.12.);
- at least one injection screw (1.13.).

2. The system according to claim 1, wherein at least two parallel implantation rods (R1, R2) are included or wherein at least two coaxial implantation rods (R1, R3) are included or wherein at least two parallel implantation rods (R1, R2; R3, R4) and at least two coaxial implantation rods (R1, R3; R2, R4) are included.

3. The system according to claim 1 or claim 2, wherein at least two coaxial implantation rods (R1, R3) are connected by means of a coaxial connector (1.11.) and/or wherein at least two parallel implantation rods (R1, R2) are connected by means of a closed parallel connector (1.10.).

4. The system according to any of the claims 1 to 3, wherein the sacrum plate (1.1.) and/or the polyaxial screw (1.2.) and/or the monoaxial expansive screw (1.3.) and/or the monoaxial screw (1.4.) and/or the hook (1.9.) are provided with a top locking system (3) comprising
- a tulip-shaped head 3.1. having an upper orifice 3.1.1. having an inner trapezoidal thread 3.1.2., an inner chamber 3.1.3. having a diameter allowing to house the implantation rod R1, R2, R3, ...., a large U-shaped indentation 3.1.6. on two opposite sides of the tulip-shaped head 3.1., a lateral slot 3.1.4. on the respective other two opposite sides outside of the tulip-shaped head 3.1., and, in the case of the tulip-shaped head 3.1. for a polyaxial screw, a lower orifice 3.1.5. smaller in diameter than the upper orifice 3.1.1. thereby forming an annular base; and
- a cylindrical threaded nut 3.2. having an upper and a lower surface 3.2.1., 3.2.2. and an outer trapezoidal thread 3.2.3. fitting with the inner trapezoidal thread 3.1.2. of the tulip-shaped head 3.1. and intended to be screwed into the inner trapezoidal thread 3.1.2. of the tulip-shaped head 3.1. so as to fix the implantation rod R1, R2, R3, ... housed in said inner chamber 3.1.3. by a contact between said implantation rod R1, R2, R3, ... and said lower surface 3.2.2. to the annular base;
- said top locking system 3 being provided on the respective sides of said sacrum plate 1.1. and/or polyaxial screw 1.2. and/or monoaxial expansive screw 1.3. and/or monoaxial screw 1.4. and/or hook 1.9. which are opposite to the respective threaded screw sides or blades thereof.

5. The system according to claim 4, wherein the top locking system (3) in polyaxial screws (1.2.) additionally comprises an intermediate element (3.3.) comprising a U-shaped part (3.3.1.) adapted to house an implantation rod (R1, R2, R3, ...) and a hemispherical part (3.3.2.) on the lower side of the U-shaped part (3.3.1.) adapted to be received by the hollow part of the head of the polyaxial screw 1.2. and to evenly transmit forces received from the U-shaped part (3.3.1.) radially.

6. The system according to claim 4 or claim 5, wherein the top locking system (3) additionally comprises a polyaxial screw (1.2.) comprising a lower threaded screw body (1.2.1.), an upper unthreaded screw body (1.2.4.) and an uppermost screw head (1.2.2.) having circumventing indentations on its lower side adapted to evenly distribute forces applied to the upper side of the spherical hollow part of the screw head (1.2.2.) radially.

7. The system according to claim 4 or claim 5, wherein the tulip-shaped head (3.1.) is directly mounted to the uppermost screw head (1.2.2.) of a polyaxial screw body (1.2.).

8. The system according to any of the claims 1 to 7, wherein said injection screw (1.13.) comprises an inner main channel (1.13.2.) extending from an opening at the top (1.13.1.) of the injection screw (1.13.) in parallel to its axis down to an area of the screw (1.13.) having lateral openings (1.13.3.), said inner main channel (1.13.2.) not extending to the lower tip of the screw (1.13.) and opening to one or more of the said openings (1.13.3.), allowing a bone cement or equivalent bone substitution material filled into the opening (1.13.1.) at the top of the screw (1.13.) to flow along the inner main channel (1.13.2.) and exiting via the lateral openings (1.13.3.) into the environment (1.13.5.) surrounding the screw (1.13.).

9. The use of a system according to any of the claims 1 to 8 for immobilizing at least parts of the spinal column of a human.

10. The use of a system according to any of the claims 1 to 8 for the manufacture of an immobilizing means for the treatment of traumatic conditions or traumas, preferably fractures, dislocations, disk herniation and numerous other injuries causing instability of the vertebra; degenerative diseases and conditions on an either acute or chronic process basis, preferably for spondylolysis; inflammatory diseases and conditions on an either acute or chronic inflammatory basis, preferably spondylarthritis, spondylitis and those diseases of rheumatic nature; tumor diseases and conditions as either benign or malign tumors; and congenital pathologies of greatly varied origin, preferably spondylolisthesis.

11. The use of claim 9 or claim 10 in cases of spinal fusion surgery, preferably in spinal fusion surgery by anterior or posterior approach.

12. The use of any of claims 9 to 11, in cases of the presence of an injection screw 1.13., for a fixation in bones in cases of osteoporosis, osteolytic neoplasm (metastasis or primary), revision surgery (screw loosening) and or rheumatic diseases with poor bone quality.

13. A method of fixing at least one implantation rod (R1, R2, R3, ...) to at least parts of the spinal column of a human, said method comprising the steps of
- selecting at least one implantation rod (R1, R2, R3, ...) suitable in diameter(s) and length(s) for the intended implantation;
- either drilling at least one hole into at least two vertebrae, or preparing the space around the laminae or vertebral pedicle, at locations allowing a fixation of said at least one implantation rod to the spinal column at a desired position;
- inserting at least one, preferably at least two, of the following means:
■ at least one sacrum plate (1.1.);
■ at least one polyaxial screw (1.2.);
■ at least one monoaxial expansive screw (1.3.);
■ at least one monoaxial screw (1.4.);
■ at least one side loading screw (1.5.2.);
■ at least one side loading expansive screw (1.7.);
■ at least one injection screw (1.13.);
- into the hole(s) drilled
- and/or fixing to the space prepared around the laminae or vertebral pedicle
■ at least one hook (1.9.)
- surrounding said pedicle or lamina; and
- fixing to any one or more of the side loading screws (1.5.2.) or side loading expansive screw (1.7.) or injection screw (1.13.) any other suitable screw at least one of
■ at least one multidirectional coupler (1.5.);
■ at least one multidirectional offset coupler (1.6.);
■ at least one multidirectional double coupler (1.12.);
- so as to allow a fixation of said at least one implantation rod (R1, R2, R3, ...) to said spinal column; and
- optionally further stabilize the arrangement of the above means by the application, between at least two of said implantation rods (R1, R2, R3, ...), of at least one of
■ at least one closed parallel connector (1.10.);
■ at least one coaxial connector (1.11.);
■ at least one transverse connector (1.8.).

14. The method according to claim 13, wherein at least two parallel implantation rods (R1, R2) are used or wherein at least two coaxial implantation rods (R1, R3) are used or wherein at least two parallel implantation rods (R1, R2; R3, R4) and at least two coaxial implantation rods (R1, R3; R2, R4) are used.

15. The method according to claim 13 or claim 14, wherein at least two coaxial implantation rods (R1, R3) are connected by means of a coaxial connector (1.11.) and/or wherein at least two parallel implantation rods (R1, R2) are connected by means of a transverse connector (1.8.).

16. The method according to any of the claims 13 to 15, including providing the sacrum plate (1.1.) and/or the polyaxial screw (1.2.) and/or the monoaxial expansive screw (1.3.) and/or the monoaxial screw (1.4.) with a top locking system (3) comprising
- a tulip-shaped head 3.1. having an upper orifice 3.1.1. having an inner trapezoidal thread 3.1.2., an inner chamber 3.1.3. having a diameter allowing to house the implantation rod R1, R2, R3, ...., a large U-shaped indentation 3.1.6. on two opposite sides of the tulip-shaped head 3.1., a lateral slot 3.1.4. on the respective other two opposite sides outside of the tulip-shaped head 3.1., and, in the case of the tulip-shaped head 3.1. for a polyaxial screw, a lower orifice 3.1.5. smaller in diameter than the upper orifice 3.1.1. thereby forming an annular base; and
- a cylindrical threaded nut 3.2. having an upper and a lower surface 3.2.1., 3.2.2. and an outer trapezoidal thread 3.2.3. fitting with the inner trapezoidal thread 3.1.2. of the tulip-shaped head 3.1. and intended to be screwed into the inner trapezoidal thread 3.1.2. of the tulip-shaped head 3.1. so as to fix the implantation rod R1, R2, R3, ... housed in said inner chamber 3.1.3. by a contact between said implantation rod R1, R2, R3, ... and said lower surface 3.2.2. to the annular base;
- said top locking system 3 being provided on the respective sides of said sacrum plate 1.1. and/or polyaxial screw 1.2. and/or monoaxial expansive screw 1.3. and/or monoaxial screw 1.4. and/or hook 1.9. which are opposite to the respective threaded screw sides or blades thereof.

17. The method according to claim 16, additionally including providing the top locking system (3) in a polyaxial screw (1.2.) with an intermediate element (3.3.) comprising a U-shaped part (3.3.1.) adapted to house an implantation rod (R1, R2, R3, ...) and a hemispherical part (3.3.2.) on the lower side of the U-shaped part (3.3.1.) adapted to be received by the hollow part of the head of the polyaxial screw 1.2. and to evenly transmit forces received from the U-shaped part (3.3.1.) radially.

18. The method according to claim 16 or claim 17, additionally including providing the top locking system (3) with a polyaxial screw (1.2.) comprising a lower threaded screw body (1.2.5.), an upper unthreaded screw body (1.2.4.) and an uppermost screw head (1.2.2.) having circumventing indentations on its lower side adapted to evenly distribute forces applied to the upper side of the screw head (1.2.2.) radially.

19. The method according to claim 16 or claim 17, additionally including mounting the tulip-shaped head (3.1.) directly to the upper unthreaded screw body (1.2.2.) of a screw.

20. The method according to any of the claims 13 to 19, wherein the at least one implantation rod (R1, R2, R3, ...) is fixed to at least parts of the spinal column of a human.

21. The method according to any of the claims 13 to 20, wherein a fixation by said injection screw(s) (1.13.) is effected by the steps of either drilling at least one hole into at least two vertebrae, or preparing the space around the laminae or vertebral pedicle, at locations allowing a fixation of said at least one implantation rod (R1, R2, R3, ...) to the spinal column at a desired position via said injection screw (1.13.); filling a bone cement or equivalent bone substitution material into the opening (1.13.1.) at the top of the screw (1.13.) and allowing it to flow along the inner main channel (1.13.2.) and exiting via the lateral openings (1.13.3.) into the environment (1.13.5.) surrounding the screw (1.13.); thereby allowing a secured fixation, via said injection screw(s) (1.13.) and the bone cement or equivalent bone substitution material in the environment (1.13.5.) of said injection screw (1.13.), of said implantation rod(s) (R1, R2, R3, ...) to the spinal column.
